(19) **Europäisches Patentamt / European Patent Office / Office européen des brevets**

(11) **EP 1 773 434 B1**

(12) **EUROPEAN PATENT SPECIFICATION**

(45) Date of publication and mention of the grant of the patent:
**16.06.2010 Bulletin 2010/24**

(51) Int Cl.:
*A61M 16/00* (2006.01)     *F04C 18/12* (2006.01)
*F04C 29/00* (2006.01)

(21) Application number: **04810758.5**

(22) Date of filing: **10.11.2004**

(86) International application number:
**PCT/US2004/037659**

(87) International publication number:
**WO 2006/022787 (02.03.2006 Gazette 2006/09)**

(54) **METHOD AND APPARATUS FOR REDUCING NOISE IN A ROOTS-TYPE BLOWER**

VERFAHREN UND GERÄT ZUR GERÄUSCHVERRINGERUNG IN EINEM GEBLÄSE VOM ROOTS TYP

PROCEDE ET APPAREIL DESTINES A REDUIRE LE BRUIT D'UN COMPRESSEUR TYPE ROOTS

(84) Designated Contracting States:
**AT BE BG CH CY CZ DE DK EE ES FI FR GB GR HU IE IS IT LI LU MC NL PL PT RO SE SI SK TR**

(30) Priority: **04.08.2004 US 912747**

(43) Date of publication of application:
**18.04.2007 Bulletin 2007/16**

(73) Proprietor: **Pulmonetic Systems, Inc.**
**Plymouth, MN 55447 (US)**

(72) Inventors:
• **DEVRIES, Douglas, F.**
**Kenmore, WA 98028 (US)**
• **WILLIAMS, Malcolm**
**San Clemente, CA 92674 (US)**

(74) Representative: **Gervasi, Gemma**
**Notarbartolo & Gervasi GmbH**
**Bavariaring 21**
**80336 München (DE)**

(56) References cited:
| DE-A1- 3 238 015 | DE-A1- 3 414 064 |
| DE-A1- 3 620 792 | GB-A- 2 157 370 |
| US-A- 5 823 186 | US-B1- 6 484 719 |

**Description**

FIELD OF THE INVENTION

The present invention relates to Roots-type blowers and, more particularly, to a method and apparatus for reducing the noise generated by such a blower.

CROSS-REFERENCE TO RELATED APPLICATION

This patent application claims the benefit of the filing date of pending U.S. Patent Application Serial No. 10/912,747, filed August 4, 2004, which claims the benefit of U.S. Provisional Patent Application Serial No. 60/492,421, filed August 3, 2003.

BACKGROUND OF THE INVENTION

Document DE 32 38 015 A1 is considered to constitute the closest prior art and discloses a Roots-type blower according to the preamble of claim 1.

Roots-type blowers have potential application in a wide variety of environments. They are relatively efficient, and can produce a wide range of delivery pressures and volumes. However, they produce a high level of noise. The highnoise level produced by Roots blowers has limited their use in environments where such high noise levels are unacceptable. One such environment is providing breathing assistance to patients by means of a mechanical ventilator.

For a variety of reasons, there are instances when individuals (patients) with acute and chronic respiratory distress cannot ventilate themselves (i.e. breathe). In those circumstances, such patients require breathing assistance to stay alive. One solution is to provide those patients with a medical device called a mechanical ventilator, which assists with their breathing.

A purpose of a mechanical ventilator is to reproduce the body's normal breathing mechanism. Most mechanical ventilators create positive intrapulmonary pressure to assist breathing. Positive intrapulmonary pressure is created by delivering gas into the patient's lungs so that positive pressure is created within the alveoli (i.e. the final branches of the respiratory tree that act as the primary gas exchange units of the lung). Thus, a mechanical ventilator is essentially a device that generates a controlled flow of gas (e.g., air or oxygen) into a patient's airways during an inhalation phase, and allows gas to flow out of the lungs during an exhalation phase.

Mechanical ventilators use various methods to facilitate precise delivery of gas to the patient. Some ventilators use an external source of pressurized gas. Other ventilators use gas compressors to generate an internal source of pressurized gas.

Most ventilator systems that have an internal gas source use either constant speed or variable speed compressors. Constant speed compressors are usually continuously operating, rotary-based machines that generate a fairly constant rate of gas flow for ultimate delivery to the patient. These constant speed systems generally use a downstream flow valve to control flow of the gas to the patient, with a bypass or relief mechanism to divert excess flow that is at any time not needed by the patient (e.g. during exhalation).

Variable speed compressors operate by rapidly accelerating from a rest state to the rotational speed needed to produce the flow rate necessary during the beginning of the inhalation phase, and then decelerating to a rest or nearly rest state at the end of the inhalation phase to allow the patient to exhale.

Two types of variable speed compressor systems are typically employed in the mechanical ventilator art: piston-based systems and rotary-based systems. An example of a prior art variable speed compressor system for use in a mechanical ventilator is described in U.S Patent No. 5,868,133 to DeVries et al. This system uses drag compressors to provide the desired inspiratory gas flow to the patient.

Rotary compressor systems deliver the required gas flow during inhalation by accelerating the compressor rotor(s) to the desired speed at the beginning of each inspiratory phase and decelerating the compressor rotor(s) to a rest or nearly rest speed at the end of each inspiratory phase. Thus, the rotary compressor is stopped, or rotated at a nominal base rotational speed, prior to commencement of each inspiratory ventilation phase. Upon commencement of an inspiratory phase, the rotary compressor is accelerated to a greater rotational speed for delivering the desired inspiratory gas flow to the patient. At the end of the inspiratory phase, the rotational speed of the compressor is decelerated to the base speed, or is stopped, until commencement of the next inspiratory ventilation phase. Prior art systems typically use a programmable controller to control the timing and rotational speed of the compressor.

Great strides have been realized in reducing the size of mechanical ventilators. Ventilators are now available that are portable, and allow users a limited degree of autonomous mobility. Further reducing the size and power requirements of mechanical ventilators hold the potential of giving patients even greater freedom of movement, enhancing their quality of life.

Because of its relative efficiency, a Roots blower can potentially contribute to the reduction in size and power consumption of mechanical ventilators. However, heretofore it has not been possible to reduce the noise created by a Roots blower to the level that is acceptable for a mechanical ventilator.

Roots blowers use a pair of interacting rotors. Each rotor has two or more lobes. The rotors are rotated inside a housing having an inlet and an outlet. The rotors rotate with the lobes of one rotor moving into and out of the spaces between the lobes of the other. Gas is moved through the blower in chambers formed by adjacent lobes of a rotor and the adjacent rotor housing wall. These chambers will be referred to herein as "gas transport chambers."

Noise is generated by roots blowers in a number of ways. One type of noise is caused by pulsing flow. As the rotors rotate, the gas transport chambers between the lobes of each rotor are sequentially exposed to the outlet. As each chamber is exposed to the outlet, a lobe of the mating rotor rotates into the chamber, displacing the gas in chamber to the outlet, causing a flow/pressure pulse. In the case of a pair of rotors each having two lobes, during each cycle of the blower, there are four pulses generated by the displacement of gas by the gas transport chambers. These pulses generate a substantial amount of noise.

A second type of noise is generated by a phenomenon known as "flow back." As each rotor rotates, it inducts gas at low pressure at the inlet. This gas is generally trapped in the gas transport chambers as the rotor moves towards the outlet. When this pocket of gas initially reaches the outlet, it is exposed to higher pressure gas at the outlet. At that time, the higher pressure gas at the outlet rushes backwardly into the gas transport chamber that contains the lower pressure gas that is being delivered from the inlet.

This reverse gas flow is very sudden. Its duration and magnitude depends on a number of factors, including the rotational speed of the rotors and the difference between the pressure of the gas in the gas transport chamber (which is typically close to the inlet pressure) and the pressure at the outlet. As a result of this sudden reverse gas flow, a pressure spike of substantial amplitude is generated. This pressure spike is generated multiple times per cycle of the blower, each time a gas transport chamber is exposed to the outlet. The resulting series of pressure spikes creates continuous noise at a level that is unacceptable for many applications, including mechanical ventilators.

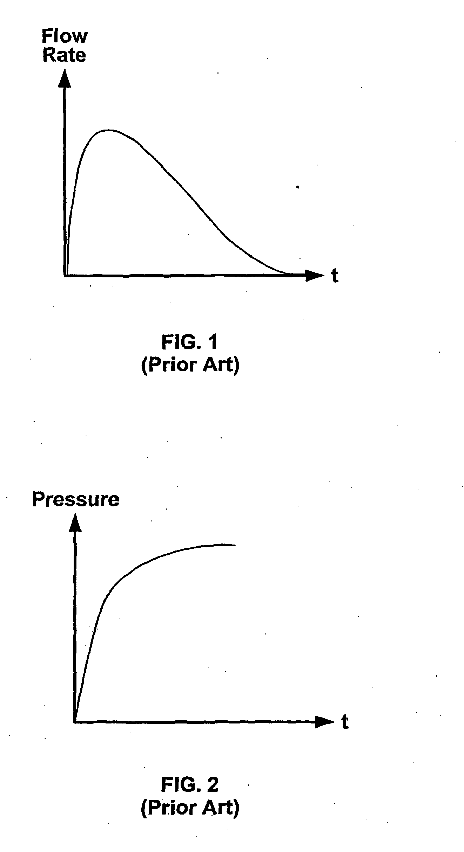

Figures 1 and 2 are diagrams that illustrate the change in gas flow back rate and associated change in gas pressure, respectively, immediately after a gas transport chamber of a Roots blower in accordance with the prior art is exposed to the outlet area. As illustrated, gas flow rate changes very abruptly with time, as does gas pressure.

Some attempts have been made to reduce the noise level of Roots blowers. To reduce the "pulsing" type of noise, the lobes of the rotors have been reconfigured so that they have a helical, rather than straight, shape. When the lobes of the rotors are straight, the gas flow into and out of the gas transport chamber is very abrupt. When the lobes are helical in shape, each lobe displaces gas over a larger angle of rotation. This spreads the displacement of gas over an angle of rotation, lessening the magnitude of the pressure pulse caused by the gas displacement, and reducing the noise created by the blower. However, this lobe design does not address the problem of flow back, since the relative pressure between the gas at the outlet and gas being delivered from the inlet is still the same.

Attempts have also been made to reduce flow back noise. Various kinds of channels or passages have been provided that allow some gas to flow from the outlet to the gas transport chamber prior to the time the chamber reaches the outlet, thereby increasing the gas pressure in the chamber and reducing the pressure spike that occurs when the gas in the chamber is exposed to the higher outlet pressure. Heretofore, however, it has not been possible to reduce the noise of a Roots blower to the extent required for use in a noise sensitive application such as a mechanical ventilator.

SUMMARY OF THE INVENTION

The invention is a method and apparatus for reducing the noise generated by a Roots-type blower. The invention has particular use in mechanical ventilators, though the advantages thereof may be realized in many different noise-sensitive blower applications.

The blower of the present invention comprises a housing defining a rotor chamber and an inlet and outlet to the rotor chamber. First and second rotors are mounted in the rotor chamber, each rotor defining a plurality of spaced lobes. Adjacent lobes of a rotor cooperate with the housing to define a series of generally closed chambers that move from the inlet to the outlet as the rotors are rotated. These chambers are referred to herein as "gas transport chambers." In one or more embodiments, the blower is configured with helical rotors as known in the art to reduce noise caused by pulsing flow.

In addition, the blower is specially configured so that the pressure within a gas transport chamber increases from the inlet pressure to the outlet pressure in a generally or approximately linear manner as the chamber approaches the outlet.

In one embodiment, the net flow rate of gas from the outlet into the gas transport chamber is regulated to control pressure change within the chamber. In one embodiment, a flow path from the outlet to the gas transport chamber and/or from the gas transport chamber to the inlet is provided. The flow path is configured such that a net flow rate of gas from the outlet into the gas transport chamber is generally or approximately constant during the time the gas transport chamber approaches the outlet, such that the resulting pressure change in the chamber is generally or approximately linear.

In one or more embodiments, the flow path comprises at least one outlet flow channel formed in the interior surface of the housing. The outlet flow channel extends from a point before the outlet (when considering the rotational direction of the rotor) to the outlet. The flow channel is configured to permit gas to flow from the outlet into a gas transport chamber while the gas transport chamber is proceeding towards the outlet. In one embodiment, the cross-sectional area of the outlet flow channel increases non-linearly and continuously moving from its first end towards the outlet to maintain an approximately constant flow rate of gas into the gas transport chamber and/or an approximately linear rate of change in pressure in the chamber. In one embodiment, an outlet flow channel is provided corresponding to each rotor.

In one embodiment, the flow path comprises at least one inlet flow channel formed in the interior surface of the housing extending from a point after the inlet (when considering the rotational direction of the rotor) to the inlet. The inlet flow channel is configured to permit gas to flow from a gas transport chamber to the inlet as the gas pressure in the gas transport chamber rises as a result of gas entering the gas transport chamber from the outlet via the outlet flow channel. In one embodiment, an inlet flow channel is provided corresponding to each rotor.

In one embodiment, both outlet and inlet flow channels are provided. The flow channels at the inlet and outlet work cooperatively to control the net flow of gas into the gas transport chamber and thereby the rate of change of pressure in the gas transport chamber.

One or more embodiments of the invention comprise a method for determining the configuration of the flow path so as to achieve a desired rate of pressure change in the gas transport chamber. In one embodiment, an initial flow channel configuration is chosen, and then a modeling process is used to determine the pressure change over time within the gas transport chamber at desired operating parameters (e.g. rotational speed, temperature, inlet and outlet pressures) using known equations that govern compressible gas flow. If the resulting pressure change over time does not match the desired result, the flow channel configuration is adjusted and the pressure change within the gas transport chamber is again determined for the modified flow channel configuration. A number of iterations may be performed until a satisfactory result is achieved. In one embodiment, a desired result is an approximately or generally linear change in the gas transport chamber pressure over time.

Alternatively, in another embodiment, instead of starting with an assumed flow channel configuration and adjusting it iteratively until a satisfactory pressure profile is achieved, a desired pressure profile may be used to directly analytically and/or numerically calculate the flow channel profile that will achieve that desired pressure profile.

Further objects, features, and advantages of the present invention over the prior art will become apparent from the detailed description of the invention which follows, when considered with the attached figures.

## DESCRIPTION OF THE DRAWINGS

FIGURE 1 is a diagram illustrating a change in gas flow back rate over time for a gas transport chamber of a Roots-type blower in accordance with the prior art;

FIGURE 2 is a diagram illustrating change in pressure over time during flow back for the prior art blower illustrated in Figure 1;

FIGURE 3 is a perspective exploded view of a Roots-type blower in accordance with an embodiment of the present invention;

FIGURE 4 is a perspective end view of a housing of the blower illustrated in Figure 3;

FIGURE 5 is an enlarged view of an outlet of the blower housing illustrated in Figure 4, as viewed from an interior of the housing;

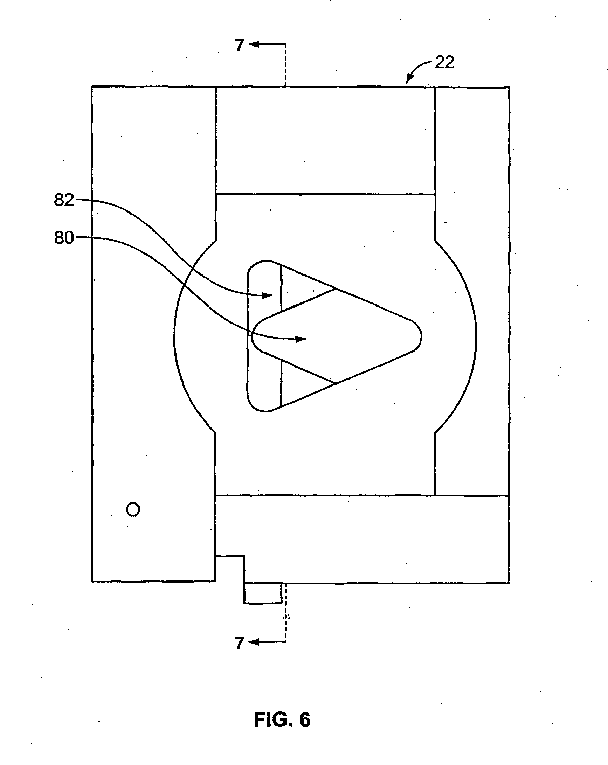

FIGURE 6 is a first side view of the housing illustrated in Figure 3, illustrating an outlet of the blower housing;

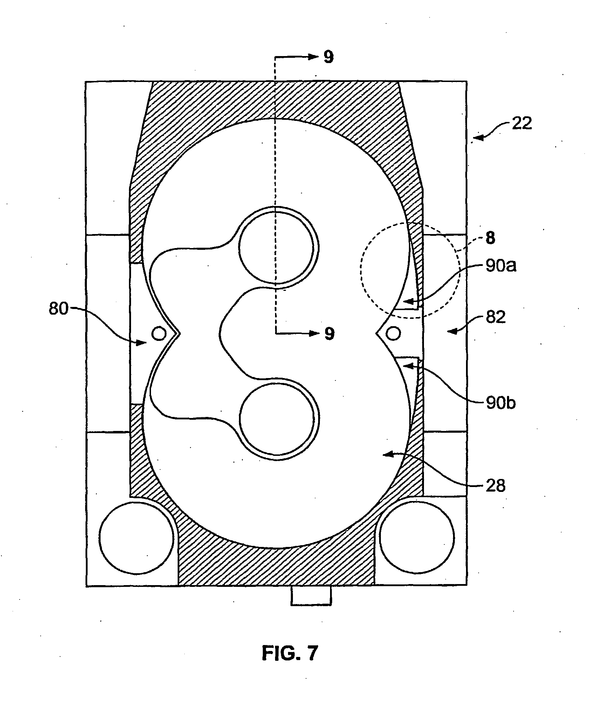

FIGURE 7 is a cross-sectional view of the housing illustrated in Figure 6 taken along line 7-7 therein;

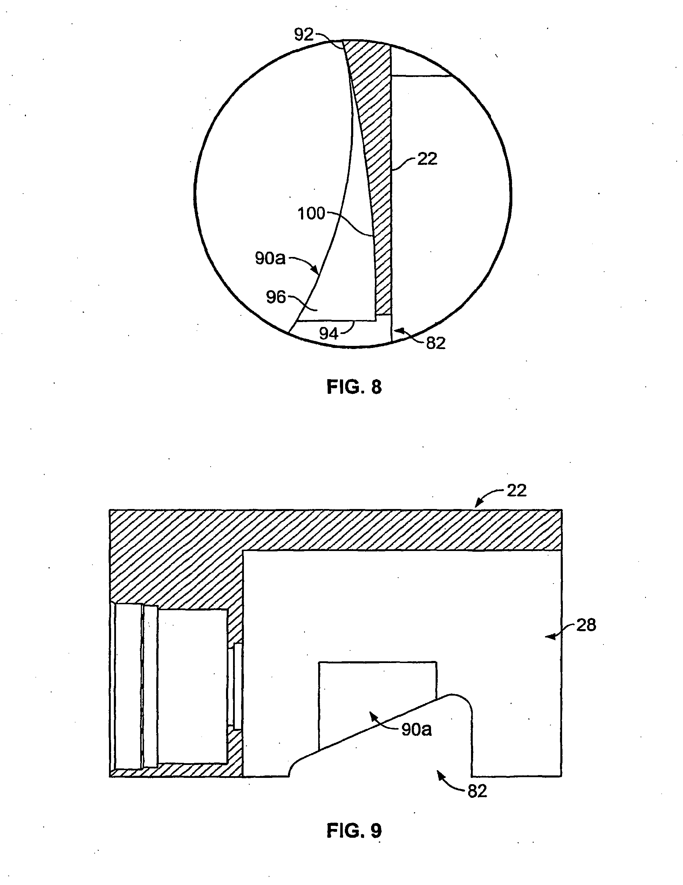

FIGURE 8 is an enlarged view of a flow channel an a portion of the outlet of the blower housing illustrated in Figure 7;

FIGURE 9 is an enlarged view of a portion of the housing illustrated in Figure 7 taken in the direction of line 9-9 therein;

FIGURE 10 is a cross-sectional view of a housing of a blower in accordance with an embodiment of the invention in which inlet and outlet flow channels are provided;

FIGURE 11 is an enlarged view of an inlet flow channel of the blower illustrated in Figure 10;

FIGURE 12 is a flow diagram illustrating steps of a first method for determining the configuration of a flow path in accordance with an embodiment of the invention;

FIGURE 13 is a flow diagram illustrating steps of a second method for determining the configuration of a flow path in accordance with an embodiment of the invention;

FIGURE 14 is a diagram illustrating changes in gas flow rate over time in accordance with a blower configured in accordance with the present invention; and

FIGURE 15 is a diagram illustrating change gas pressure over time in accordance with a blower configured in accordance with the present invention.

## DETAILED DESCRIPTION OF THE INVENTION

The invention is a method and apparatus for reducing Roots-type blower noise. In the following description, numerous specific details are set forth in order to provide a more thorough description of the present invention. It will be apparent, however, to one skilled in the art, that the present invention may be practiced without these specific details. In other instances, well-known features have not been described in detail so as not to obscure the invention.

In general, the invention comprises a Roots-type blower. The blower includes two inter-meshing rotors which rotate within a housing. The rotors draw gas from an inlet and deliver it through the housing to the outlet. The rotors each have two or more lobes. Adjacent lobes on each rotor, in combination with the housing, define gas transport chambers that transport the gas from the inlet to the outlet.

In one embodiment, the blower includes one or more flow channels that define flow paths permitting gas to flow from the outlet to the gas transport chambers. In one embodiment, one or more of such outlet flow channels are provided corresponding to each of the rotors.

In one embodiment, the blower additionally includes one or more flow channels that define flow paths permitting gas to flow from the gas transport chambers to the inlet. In one embodiment, one or more of such inlet flow channels are provided corresponding to each of the rotors. In one embodiment, flow channels are provided at both the inlet and outlet.

In one or more embodiments, one or more flow channels are configured to regulate the net rate of gas "flow back" into a gas transport chamber such that the gas flow rate is generally or approximately constant, and/or such that changes in gas pressure within the chamber are generally or approximately linear. The configuration of the invention thus reduces pressure spikes associated with gas flow back, thus substantially reducing the noise generated by the blower.

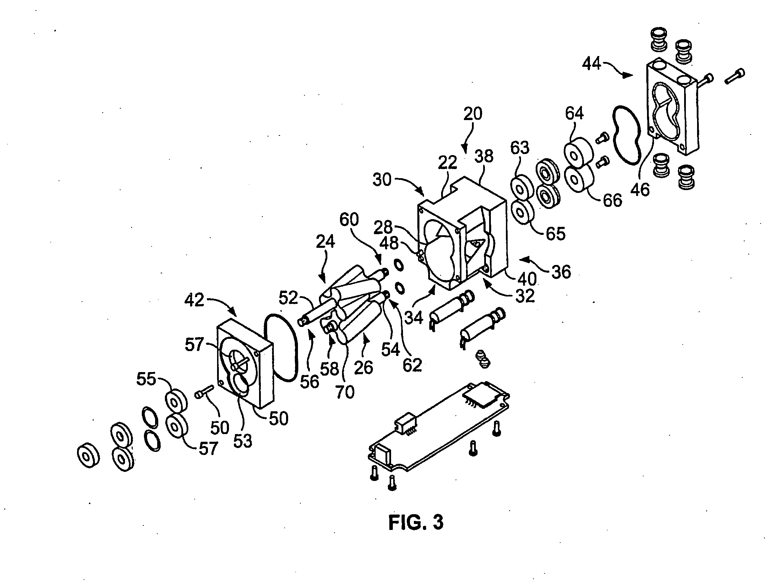

Figure 3 shows a Roots-type blower 20 in accordance with an embodiment of the invention. As illustrated, the blower 20 comprises a housing 22, a first rotor 24, and a second rotor 26.

As described in more detail below, the housing 22 may have a variety of configurations. As illustrated, the housing 22 comprises a casing defining a rotor chamber 28. As illustrated, the chamber 28 has the configuration of two intersecting cylinders.

In one embodiment, the casing is a walled structure. The external shape of the casing may vary. In one embodiment, it is generally cube-shaped. In that configuration, the housing 22 has a first side 30 and a generally opposing second side 32. The housing 22 has a first end 34 and a generally opposing second end 36, and a top 38 and a bottom 40.

In one embodiment, the chamber 28 has a longitudinal axis which extends through the first and second ends 34,36 of the housing 22. In one embodiment, the first end 34, of the housing is open, while the second end 36 of the housing 22 is closed. This permits the rotors 24,26 to be inserted into and removed from the housing 22 via the first end 34.

In this embodiment, a first end plate 42 is used to close the first end 34 of the housing 22. A cover plate 44 is located at the second end 36 of the housing 22. As illustrated, the first end plate 42 has recessed portions 51 and 53 for accepting bearings 55 and 57, respectively. The cover plate 44 has an inset or recessed portion 46 for accepting gears 64 and 66 that are mounted on second ends 60 and 62 of shafts 50 and 52, respectively, that protrude through bores in second end 36 of housing 22 when rotors 24 and 26 are mounted on shafts 50 and 52 and inserted into chamber 28. In the embodiment of Figure 3, gears 64,66 are supported in a driving relationship by bearings 63 and 65 mounted in appropriate insets or reset portions in the second end 36 of the housing 22 (not shown) that are similar to insets or reset portions 51 and 53 in first end plate 42. In operation, gears 64 and 66 are protected and covered by the cover plate 44, being located in the recess or inset 46 thereof. Gears 64 and 66 intermesh with one another and insure that, in operation, rotors 24 and 26 rotate in proper relationship to one another so that their respective lobes 70 mesh but do not actually touch.

The first end plate 42 and the cover plate 44 are preferably removably connectable to the housing 22. The first end plate 42 and the cover plate 44 may be connected to the housing 22 using one or more fasteners. In one embodiment, one or more pins 48 extend from the first end 34 of the housing 22 for insertion into mating apertures in the first end plate 42. These pins 48 aid in maintaining the first end plate 42 in aligned connection with the housing 22. One or more threaded fasteners 50 are extended through the first end plate 42 into engagement with the housing 22, thereby connecting or fastening the first end plate 42 to the housing 22. The cover plate 44 is preferably similarly connected to the housing 22.

In the embodiment illustrated, the first rotor 24 is mounted on a first shaft 52 and the second rotor 26 is mounted on a second shaft 54. Alternatively, the rotors 24 and 26 may be integrally formed with shafts 52 and 54, respectively. In the embodiment illustrated, a first end 56 of the first shaft 52 extends through the first end plate 42. Means are provided for driving the first shaft 52. This means may comprise, for example, a brushless DC electric motor. One embodiment of such an electric motor is described in U.S. Patent Application Serial No.10/847,693 filed May 18, 2004, owned by the same assignee hereof. Of course, the means for driving the first shaft 52 may comprise a variety of elements. Further, the means by which the first shaft 52 is driven by that means may vary, such as by direct drive or indirect drive.

In one embodiment, a first end 58 of the second shaft 54 is supported for rotation by the first end plate 42. This may be accomplished by bearing 57 or similar means.

It will be appreciated that the rotors 24,26 may be driven by means of a variety of other drive configurations than as specifically illustrated and described above. For example, each of rotors 24,26 may be independently driven by separate but synchronized electric motors, or the rotors could be arranged in a driving relationship with one another in other fashions.

In one or more embodiments, except for an inlet and an outlet, the housing 22 is generally sealed so that gas enters and exits only through the inlet and outlet. In the embodiment illustrated, the first and second ends 34,36 of the housing 22 are thus sealed. Thus, in the embodiment illustrated, the first end plate 42 is sealed to the housing 22. Any of a variety of seals, bushings and the like, as known in the art, may be used to seal the connection of the first end plate 42 to the housing 22 and various of the other component connections, such as at the interface of shafts and the housing 22 and the first end plate 42.

Of course, the housing 22 may have a variety of other configurations, and other approaches and/or components for sealing the housing can be used. For example, the second end 36 of the housing 22 might also be open and then closed or covered with and end plate, or the first end 34 of the housing 22 might be closed and the second end 36 might be open.

The first and second rotors 24,26 preferably have two or more lobes 70. In a preferred embodiment, each rotor 24,26 has three lobes 70. The rotors 24,26 could have, however, as few as two and as many as four or more lobes. The lobes 70 preferably follow a helical path around the axis of shaft 52 or 54, respectively. In one embodiment, first and second ends of each lobe 70 are offset from one another by about sixty degrees (60°) radially about the rotor/shaft.

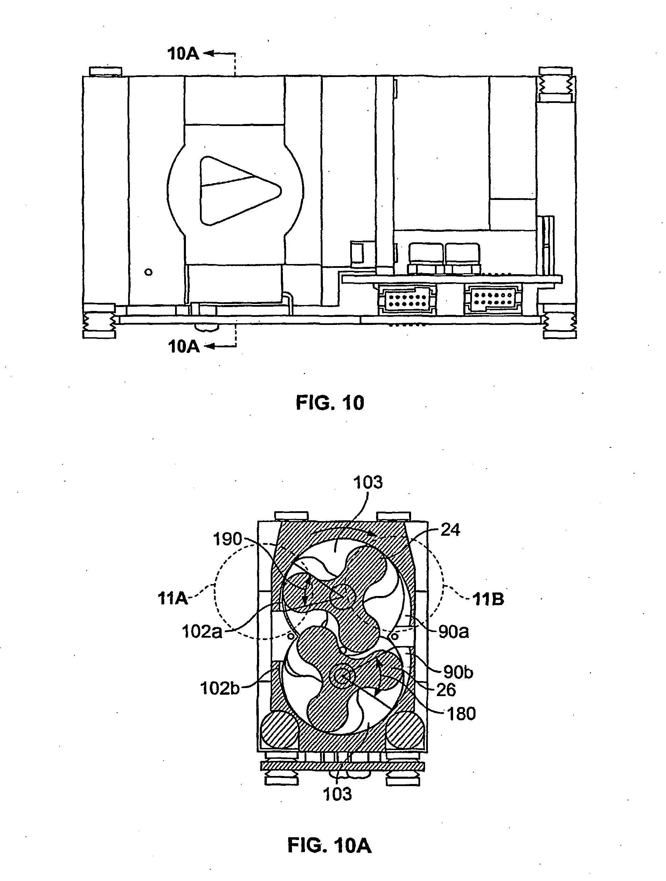

The lobes 70 extend outwardly from a center of each rotor 24,26. A space is defined between adjacent lobes 70. As is known, the lobes 70 of one rotor 24,26 are configured to mesh or engaged with the other rotor by entering the space defined between adjacent lobes of the other rotor. When the rotors 24,26 are mounted in the housing 22, the adjacent lobes 70 of each rotor 24,26, in cooperation with the interior of the housing 22, as shown in Figure 10, define gas transport chambers 103 configured to transport gas from the inlet to the outlet.

As illustrated in Figure 3, the first and second rotors 24,26 are mounted in the rotor chamber 28. The rotors 24,26 are mounted so that their shafts 52,54 extend parallel to one another and perpendicular to the first and second ends 34,36 of the housing 22.

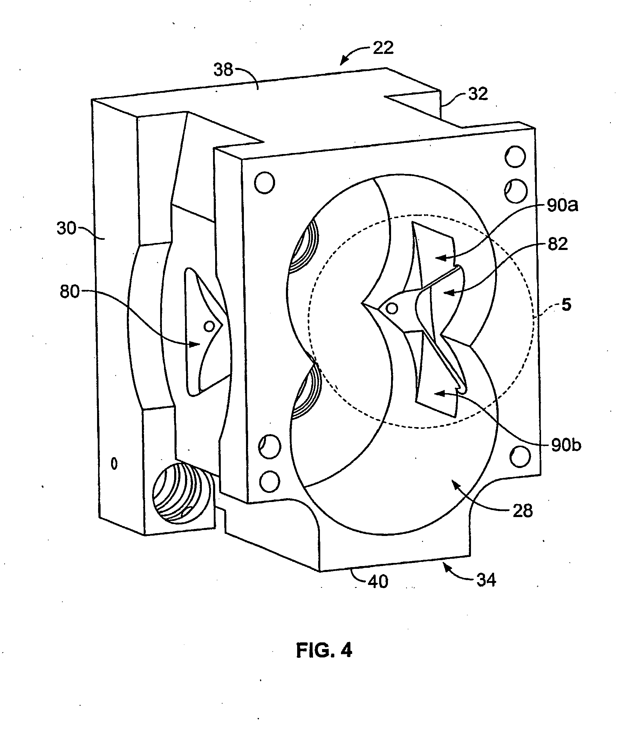

The blower 20 has an inlet through which gas is drawn and an outlet through which gas is expelled. As illustrated in Figure 4, an inlet 80 is located in the first side 30 of the housing 22. Gas is delivered to the inlet 80 of the housing 22. In one embodiment, the inlet 80 may open directly to the atmosphere. In another embodiment, a gas delivery path, such as a gas delivery tube, may define a gas flow path to the inlet 80.

An outlet 82 is formed in the second side 32 of the housing 22. As described in more detail below, gas is delivered by the rotors 24,26 from the inlet 80 to the outlet 82. The gas which is delivered to the outlet 82 by the gas transport chambers is expelled from the housing 22 through the outlet 82.

In one or more embodiments, the inlet 80 and outlet 82 are generally triangular in shape. This configuration is particularly applicable when the rotors 24,26 have helical lobes. In particular, when the lobes of the rotors 24,26 are helical, the respective tops of the inlet 80 and outlet 82 preferably slope downwardly at a similar angle as the lobes of the top rotor 24, and the respective bottoms of the inlet 80 and outlet 82 preferably slope upwardly at a similar angle as the lobes of the bottom rotor 26.

Of course, the configuration of the inlet 80 and outlet 82 may vary, particularly when the configuration of the rotors 24,26 varies. For example, if the rotors 24,26 have straight lobes, then the inlet 80 and outlet 82 might be rectangular in shape.

As described to this point, the rotors 24,26 are rotated in the housing 22 by a drive element such as a brushless DC motor. As the rotors 24,26 rotate, they deliver gas from the inlet 80 to the outlet 82. Gas is delivered in the gas transport chambers 103 situated between a "front" lobe and a "rear" lobe as shown in Figure 10. As the "front" lobe of a chamber passes the inlet, the gas transport chamber is exposed to the inlet and filled with gas at the inlet pressure. As the rotor continues to rotate, the "rear" lobe passes the inlet. At this time, the gas transport chamber is generally enclosed by the front and rear lobes and the interior surface of the housing, and is not directly exposed to either the inlet or outlet.

As the rotor continues to rotate, the leading or front lobe reaches the outlet. As the gas transport chamber is exposed to the outlet, gas initially rushes from the (higher pressure) outlet into the gas transport chamber. This "flow back" creates an undesirable pressure spike. As the rotor continues to rotate, the corresponding lobe of the second rotor rotates into the gas transport chamber, displacing the gas therein to the outlet, thereby delivering the gas from the inlet to the outlet. The rotor then rotates farther, with the gas transport chamber eventually rotating back to the inlet.

In accordance with the invention, the blower 20 is configured so that the rate of the gas flow back into the gas transport chamber is generally or approximately constant, and/or so that the rate of change of gas pressure in the gas transport chamber is generally linear as the gas transport chamber approaches the outlet. In one or more embodiments this is accomplished with one or more gas flow channels incorporated in the rotor housing. In one embodiment of the invention, flow back is controlled using one or more gas flow channels that extend from the outlet to the interior of the housing and, in a preferred embodiment, with one or more gas flow channels leading from the interior of the housing to the inlet.

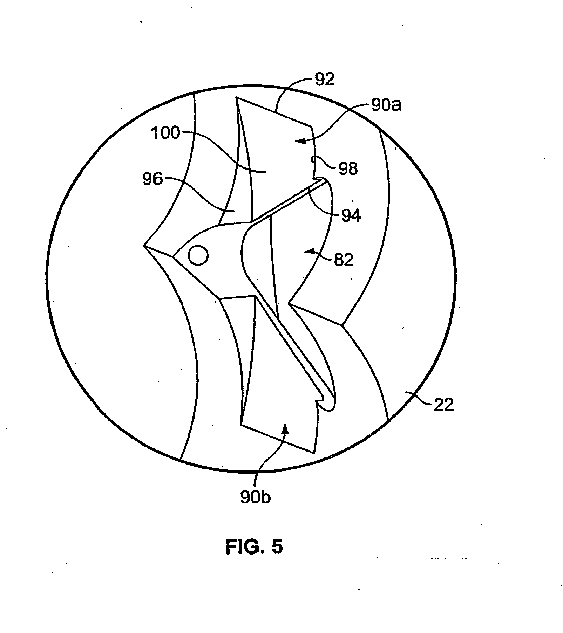

As best illustrated in Figures 4 and 5, in one embodiment, a gas flow channel is provided corresponding to each rotor 24,26, the flow channel extending from the outlet 82 into the rotor chamber 28 of the housing 22 (such a flow channel is referred to herein as an "outlet" flow channel). In one embodiment, a first outlet flow channel 90a extends from the outlet 82 in a first circumferential direction, that channel cooperating with the first rotor 24, and a second outlet flow channel 90b extends from the outlet 82 in a second circumferential direction, that channel cooperating with the second rotor 26. One of the outlet flow channels 90a will now be described in greater detail, it being understood that in a preferred embodiment, the channel 90b corresponding to the other rotor is similar.

As described in detail above, the rotors 24,26 are configured to rotate within the rotor chamber 28 defined by the housing 22. In the embodiment illustrated in Figures 4 and 5, one rotor 24 is mounted above the other rotor 26. In this embodiment, the top rotor 24 rotates in a clock-wise direction in the view of the housing 22 illustrated in Figures 4 and 5. In other words, when the rotor 24 is mounted in the housing 22, each lobe 70 thereof is moving downwardly as it sweeps towards the outlet 82.

Referring still to Figures 4 and 5, the outlet flow channel 90a begins at a point along the interior of the housing 22 before the outlet 82, when considering the rotational direction of the lobes 70 of the rotor 24. As illustrated in Figure 5, the outlet flow channel 90a has a first end 92, a second end 94, and a pair of sides 96,98. The second end 94 of the outlet flow channel 90a preferably terminates at the outlet 82. The first end 92 is, as described, located along the housing 22 at a point before the outlet 82 when considering the rotational direction of the lobes 70.

In one or more embodiments of the invention, the rotors 24,26 of the blower 20 have a maximum exterior dimension. The outer-most dimension is defined by the tips, or "lands," of the lobes 70. The rotor chamber 28 is generally configured so that the rotors 24,26 fit within the chamber 28 in close tolerance. In one embodiment, this tolerance results in a clearance spacing between the interior surface of the housing 22 and the lobes 70 of the rotors 24,26 of about .003 inches. The actual spacing may vary. As will become more apparent below, the size and configuration of the flow channels may vary depending upon this clearance spacing.

In one or more embodiments, the depth of the outlet flow channel 90a increases moving in a circumferential direction from the first end 92 towards the second end 94. The sides or side walls 96,98 of the channel 90a extend along the length of the outlet flow channel 90a from its first end 92 to its second end 94. The width of the outlet flow channel 90a is defined by the distance between the side walls 96,98. In one or more embodiments, the depth of the outlet flow channel 90a increases in a generally continuous, non-linear manner, while the width stays the same.

The depth of the outlet flow channel 90a corresponds to the height of the side walls 96,98 at a particular location along their length. As illustrated, each side wall 96,98 extends upwardly from a bottom surface 100. Each side wall 96,98 terminates at the interior surface of the housing 22. In the embodiment of Figure 5, because of the roughly triangular shape of outlet 82, side wall 98 is shorter in length than side wall 96.

As described above, outlet flow channel 90a is configured to permit gas to flow from the outlet 82 into a gas transport chamber as that gas transport chamber approaches the outlet 82. The configuration of the outlet flow channel 90a, including its size and shape, is preferably selected so that the rate of gas flow into the gas transport chamber as the gas transport chamber approaches the outlet 82 produces an approximately linear increase in pressure in the gas transport chamber over time. The rate of change in gas pressure within the gas transport chamber is generally related to the rate of gas flow into the gas transport chamber. The rate of gas flow into the gas transport chamber is generally related to the pressure difference between the outlet and the gas transport chamber, and the cross-sectional area of the outlet flow channel 90a at the point at which "front" lobe 70 of that gas transport chamber is located at any point in time. That cross-sectional area is a generally quadrilateral-shaped area formed on one side by the radially outward edge of lobe 70 and on the other three sides by the three sides of outlet flow channel 90a. In one embodiment, it has been found that increasing the area of the outlet flow channel 90a continuously and non-linearly achieves a generally or approximately constant gas flow rate into the gas transport chamber, and thus an associated generally or approximately linear rate of change of pressure, within the gas transport chamber. In particular, the cross-sectional area of the outlet flow channel 90a preferably increases continuously and non-linearly moving from the first end 92 of the channel 90a towards the outlet 82.

In operation, as a gas transport chamber of the rotor 24 passes the inlet 80, it is filled with gas at the ambient pressure at inlet 80. The ambient pressure at inlet 80 is generally lower than the outlet pressure at outlet 82. As the rotor 24 rotates and the gas transport chamber reaches the first end 92 of the channel 90a, higher pressure gas from the outlet 82 begins to flow into the gas transport chamber. At this time, the pressure difference between the gas at the outlet and the gas in the gas transport chamber is at its maximum value. Because the gas flow rate into the gas transport chamber is dependent on this pressure difference, to achieve a generally linear increase in pressure over time, the size of the channel 90a at end 92 is at a minimum.

As the rotor 24 continues to rotate towards the outlet 82, the pressure of the gas in the gas transport chamber begins to rise due to the flow of gas though channel 90a into the gas transport chamber. Because the pressure difference between the gas transport chamber and outlet 82 drops, the size of the channel 90a is increased to provide a larger cross-sectional flow area to maintain an approximately constant gas flow rate into the gas transport chamber, and thus achieve an approximately linear increase in pressure in the gas transport chamber.

Eventually, the front lobe reaches the outlet 82 and the gas transport chamber is directly exposed to the outlet 82. Because the pressure of the gas in the gas transport chamber and at the outlet have already substantially equalized, there is no abrupt pressure change, and noise is substantially reduced.

As the rotor 24 continues to rotate, a mating lobe 70 of the other rotor 26 begins to fill the gas transport chamber, displacing the gas therein out to the outlet 82.

Operation of the blower 20 with respect to the other rotor 26 is similar, with gas permitted to flow back from the outlet 82 into a gas transport chamber between lobes 70 of the rotor 26 via the outlet flow channel 90b.

In one or more embodiments of the invention, and as illustrated in Figures 10-11, the net flow rate of gas into the gas transport chambers and the resulting pressure changes are preferably further controlled by providing one or more inlet flow channels 102a,b. These inlet flow channels 102a,b define a flow path permitting gas within the gas transport chambers of the rotors 24,26 to flow back towards the inlet 80. As described above, appropriately configured outlet flow channels 90a,b are effective in creating a generally or approximately constant gas flow rate into a gas transport chamber as the gas transport chamber approaches the outlet 82, thus producing a generally or approximately linear change in gas pressure in the gas transport chamber. It has been determined, however, that when the flow path from the gas transport chamber to the inlet is also provided, the ability to control the net flow rate into the gas transport chambers, and thus the change in gas pressure in the gas transport chambers, may be further enhanced.

As such, in a preferred embodiment of the invention, flow or relief channels or passages 102a,b similar to the outlet flow channels 90a,b described above, are located at the inlet 80 (and are thus referred to herein as "inlet" flow channels). Preferably, an inlet flow channel 102a,b is provided in the housing 22 corresponding to each rotor 24,26. Inlet flow channels 102a,b are used to control the rate at which gas flows back or "leaks" from a gas transport chamber to the inlet.

To permit the rotors 24,26 to rotate within the housing 22, there must be some clearance between the "land," or outermost portion, of each lobe, and the adjacent housing wall. This small clearance results in leakage from the outlet into the gas transport chamber (via the clearance area between the "front" lobe of the gas transport chamber and the housing wall) and from the gas transport chamber back to the inlet (via the clearance area between the "back" lobe of the gas transport chamber and the housing wall). It will thus be appreciated that selection of a particular lobe clearance has an effect on the net flow of gas into the gas transport chamber.

In one embodiment, the configuration of the inlet flow channels 102a,b is selected, in conjunction with the outlet flow channels 90a,b at the outlet 80 and the inherent leakage resulting from the lobe clearance and, so that the net gas flow rate into the gas transport chamber is generally or approximately constant and/or the change in gas pressure in the gas transport chamber is generally or approximately linear as the gas transport chamber approaches outlet 82. The inlet flow channels 102a,b may have a variety of configurations. In one embodiment, the inlet flow channels 102a,b have a similar configuration to the outlet flow channels 90a,b. Specific methods for determining the configuration of the inlet and outlet flow channels are described in greater detail below.

A variety of variations of the invention are contemplated. One or more outlet and/or inlet flow channels or passages are preferably provided for both rotors. It is possible, however, to provide flow channels or passages for only one rotor.

As described, the flow channels or passages are preferably configured to result in a generally or approximately constant gas flow rate into a gas transport chamber, and thus to create a generally or approximately linear change in pressure in the gas transport chambers as the gas transport chambers approach outlet 82. The terms "generally" or "approximately" contemplate some deviation from an exact achievement of the desired goal. In one embodiment, the results achieved deviate by no more than about 30%, preferably no more than about 20%, and most preferably no more than about 5%-10% from the desired results.

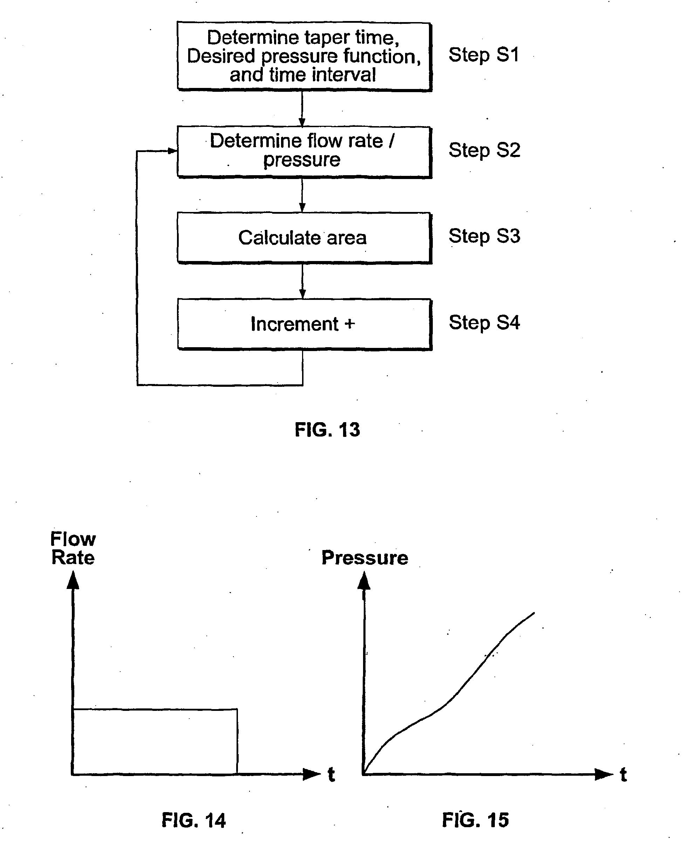

Figures 14 and 15 illustrate the net flow rate of gas into a gas transport chamber and the resulting change in pressure, over time, of one embodiment of a blower in accordance with the present invention. As illustrated in Figure 14, the flow rate is generally and approximately constant over a period of time t. As illustrated in Figure 15, the resulting change in pressure is generally or approximately linear.

One or more embodiments of the invention comprise methods of determining the configuration of the flow channels to generally or approximately achieve the desired flow/pressure characteristics. One embodiment of the invention is a method for determining the change in pressure in a gas transport chamber versus time based upon a number of variables, including an assumed flow channel profile. The method may be performed iteratively. For example, the assumed flow channel profile may be varied until a satisfactory pressure change profile is achieved.

In one embodiment, an iterative method of determining the configuration of outlet and inlet flow channels of the blower is performed by modeling the blower on a computing device. In a preferred embodiment, modeling is performed using VisSim software available from Visual Solutions, Incorporated of Westford, Massachusetts, USA. The method could, however, be done manually or using other appropriate software. The method could also be accomplished physically by building models and measuring data from use of the models.

As described in more detail below, in one method of the invention a variety of assumed or selected parameters or variables relating to or associated with the configuration of a blower (such as size/shape of the flow channels and operating parameters such inlet/outlet pressures, temperatures, delivery rates, and rotor speed) are used to calculate changes in pressure in the gas transport chambers over time, or to calculate other values of characteristics of the gas in the gas transport chamber, such as, for example, a flow rate of gas into the chamber.

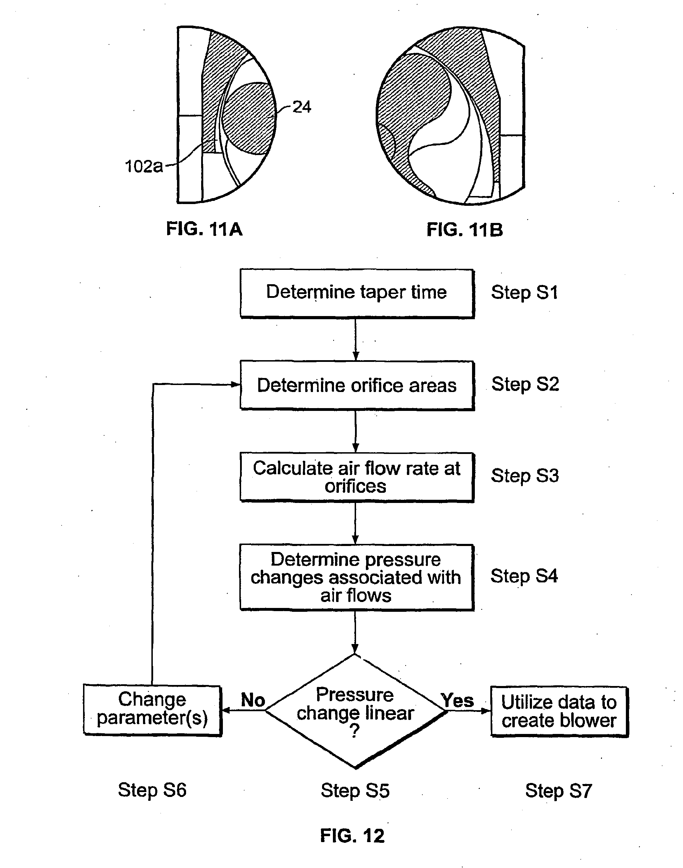

In accordance with one method of the invention, the selected parameters and/or variables are utilized to calculate or determine changes in pressure over time in the gas transport chambers of the blower as the rotors of the blower rotate based on compressible flow equations, as are known in the art. Steps in accordance with one embodiment of a method of invention are illustrated in Figure 12 and are described in more detail below.

In a first step S1, the length of time that it takes for a lobe of a rotor to traverse the angle ("taper angle") over which the desired pressure compensation of the gas transport chamber is to be accomplished is determined. This time is referred to herein as the "taper time." The taper time depends on the taper angle and the rotational speed of the rotor. In embodiments in which both outlet and inlet flow channels are used, there may be separate taper angles, and therefore taper times, for the outlet and inlet flow channels, respectively. For example, in the embodiment of Figure 10, taper angle 180 is applicable to the outlet flow channel 90b, while taper angle 190 is applicable to inlet flow channel 102b. Alternatively, a single taper angle may be applicable to both the outlet and inlet flow channels.

Thus, in one embodiment, the "taper time" is determined from an assumed operating rotational speed (measured in rpm) of the rotors and the applicable taper angle. In one embodiment, the taper time is calculated from the taper angle and the rotational speed of the rotor as follows:

$$\text{Taper time} = (1/(rpm/60)) * (\text{taper angle}/360 \quad)$$

If the blower is intended to be used over a range of rotational speeds, there will be a different taper time applicable to each rotational speed. The method may be performed at a variety of operating speeds within the operating range to select a flow channel profile that provides the most satisfactory pressure change profiles over the operating range.

After the taper time is determined, at step S2, the inlet and outlet cross-sectional flow areas (also referred to herein as "inlet orifice area" and "outlet orifice area," respectively) as a function of rotor position are determined using an assumed flow channel profile. The orifice areas may alternatively be represented as functions of the normalized taper time instead of as functions of rotor position. That is, they may be represented as functions of "t", where "t" equals the period of time from the time at which a lobe of a gas transport chamber begins to traverse the taper angle, divided by the taper time. For example, "t" will equal zero (0) when the lobe is at the beginning the taper angle, and "t" equals one (1) when the lobe reaches the end of the taper angle.

As indicated above, the total orifice area through which gas flows from the outlet to the gas transport chamber (in the case of an outlet flow channel) or from the gas transport chamber to the inlet (in the case of an inlet flow channel) at any point in time as the rotor rotates is the sum of the cross-sectional area of the flow channel at the point at which the tip (or "land") of the applicable rotor lobe is located at that time plus the cross-sectional area of the clearance gap between the lobe and the housing (the "leakage area").

In one embodiment, the depth of each flow channel varies along its length, getting deeper closer to the outlet (in the case of the outlet flow channel) or to the inlet (in the case of the inlet flow channel), while its width remains fixed. As part of one embodiment of the iterative method of the invention, an initial area profile of each flow channel is assumed, and then the resulting rate of change of pressure is calculated. Adjustments to the assumed profile are made, and then the rate of change of pressure is again calculated. This iterative process is followed until a satisfactory rate of change of pressure over the desired operating range of the blower is achieved.

In one or more embodiments, profiles for the areas of the outlet and inlet flow channels are assumed to be in the form of higher order polynomials. In one or more embodiments, the total outlet flow orifice area (including the leakage area) is assumed to have the form:

$$A_O(t) = Et^4 + Ft^7 + Gt^{12} + L$$

In the above equation, "$A_O(t)$" is the cross-sectional area of the total outlet orifice area (the sum of the outlet flow channel cross-sectional area and the leakage area) as a function of the normalized taper time "t" (that varies from 0 to 1) E, F and G are constants, and L is the leakage area. In one embodiment, values of 044 cm$^2$ (.007 in.$^2$), .126 cm$^2$ (.02 in.$^2$), and .044 cm$^2$ (.007 in.$^2$) are selected as values of constants E, F and G respectively.

In one or more embodiments, the width of the outlet flow channel is assumed to be fixed, and the depth of the outlet flow channel at any location along the outlet flow channel will be equal to the outlet flow channel cross-sectional area divided by that width.

In one or more embodiments, the outlet flow channel cross-sectional area is equal to the total outlet orifice area minus the leakage area:

$$A_{OC}(t) = A_O(t) - L = Et^4 + Ft^7 + Gt^{12}$$

Thus, the depth of the outlet flow channel as a function of the normalized taper time has the form:

$$D_O(t) = A_{OC}(t)/W_O -= (Et^4 + Ft^7 + Gt^{12})/W_O$$

Where $D_O(t)$ is the outlet flow channel depth as a function of "t," "$A_{OC}(t)$" is the outlet flow channel area as a function of "t", "$W_O$" is the outlet flow channel width, and E, F and G are constants. In one or more embodiments, "$W_O$" is the width of the outlet flow channel as measured across the land of a lobe as it traverses the outlet flow channel.

In one or more embodiments, the total inlet flow orifice area (inlet flow channel cross-sectional area plus leakage area) is assumed to have the form:

$$A_I(t) = H(1-t)^4 + I(1-t)^7 + J(1-t)^{12} + L$$

In the above equation, "$A_I(t)$" is the total inlet flow orifice area as a function of the normalized taper time "t" (that varies from 0 to 1), and H, I, and J are constants. In one embodiment, values of .006 cm$^2$ (.001 in.$^2$), 0, and .006 cm.$^2$ (.001 in.$^2$) are selected as values of constants H, I, and J, respectively.

In one or more embodiments, the width of the inlet flow channel is assumed to be fixed, and the depth of the inlet flow channel at any location along the inlet flow channel will be equal to the inlet flow channel cross-sectional area divided by that width.

In one or more embodiments, the inlet flow channel cross-sectional area is equal to the total inlet orifice area minus the leakage area:

$$A_{IC}(t) = A_I(t) - L = Ht^4 + It^7 + Jt^{12}$$

Thus, the depth of the inlet flow channel as a function of the normalized taper time has the form:

$$D_I(t) = A_{IC}(t)/W_I = (Ht^4 + It^7 + Jt^{12})/W_I$$

Where $D_I(t)$ is the inlet flow channel depth as a function of "t," "$A_{IC}(t)$" is the inlet flow channel area as a function of "t", "$W_I$" is the inlet flow channel width, and H, I and J are constants. In one or more embodiments, "$W_I$" is the width of the inlte flow channel as measured across the land of a lobe as it traverses the inlet flow channel.

In a third step S3, the flow rate of gas through the inlet and outlet orifice areas is determined as a function of time "t" based on the size of the orifice areas and pressure differences between the gas transport chamber and the inlet and outlet. In particular, the flow rate of gas from the outlet through the outlet orifice area back into the gas transport chamber ("$Q_{In}$"), and the flow rate of gas out of the gas transport chamber through the inlet orifice area back towards the inlet ("$Q_{Out}$") are determined. In one or more embodiments, $Q_{In}$ and $Q_{Out}$ are determined using compressible gas flow equations as are known in the art. For example, the equations set forth in J. D. Anderson, The Analysis & Design of Pneumatic Systems, published by Krieger Publishing Co. may be used. The total net flow rate into the gas transport chamber is the difference between these two flow rates.

$$Q = Q_{In} - Q_{Out}$$

In a step S4, the gas transport chamber pressure as a function of the normalized taper time is determined from the net flow of gas into the chamber and dead space compliance of the chamber. In this manner, it can be determined whether the pressure varies undesirably. The pressure may be analytically or numerically determined using well known principals and equations governing gas flow though orifices and into chambers. In one or more embodiments, a graph may be generated, the graph indicating pressure with respect to time.

In one or more embodiments, the pressure P in the gas transport chamber is calculated using the following equation:

$$P(t) = \int_0^t [Q/60]dt \times 1/C$$

where P is the gas transport chamber pressure, Q is the net flow rate into the gas transport chamber, and C is the dead space compliance of the gas transport chamber. In one embodiment, P is in cm $H_2 0$, Q is in liters per minute and C = . 00000167 liter/cm $H_2 0$.

As indicated, in one application of the method of the invention, it is desired that the change in pressure of gas in a gas transport chamber is generally or approximately linear as the gas transport chamber approaches the outlet. At step S5, a determination is made as to whether the rate of change in pressure determined at step S4 is sufficiently linear for the purposes for which the blower is to be used. If it is determined at step S5 that the rate of pressure change is not as linear as desired, then the outlet and/or inlet area function(s) may be modified at step S6 (e.g., by changing the flow channel depth profile by changing coefficients, taper angle or form of the function) to attempt to formulate an area function that will result in a more linear rate of pressure change. Steps S2 through S5 may then be repeated to determine whether the modified area function achieves a more linear result. Once it is determined at step S5 that the result is satisfactory, the area function and/or flow channel profile that produces that result is utilized in fabrication of the blower at step S7.

In one or more embodiments, a characteristic of the gas in the gas transport chamber other than the pressure may be of interest. For example, instead of having a desired relationship between pressure and taper time, a desired relationship may be specified between the rate of flow of gas into the gas transport chamber and the taper time. This desired relationship between the values of a characteristic of the gas in the gas transport chamber (e.g. pressure or flow rate) may be referred to as a "desired gas transport chamber function." Whatever the desired gas transport chamber function is, the iterative method of the invention may be performed until the difference between the estimated or projected values for the characteristic in question (calculated using the assumed area function) and the desired gas chamber function are satisfactory.

In accordance with the embodiment of the method as described, in one or more embodiments, both inlet and outlet flow channels are used to achieve the desired relationship (e.g. generally or approximately linear pressure change in a gas transport chamber or generally or approximately constant flow rate of gas into the gas transport chamber). Alternatively, the method can be performed using only an inlet flow channel or outlet flow channel.

In one application of the method of Figure 12, for a roots blower having rotor dimensions of 2.5 cm (1.0 in.) length and 2.2 cm (0.88 in.) diameter and operating at an inlet pressure of zero gauge pressure and an outlet pressure of 40 cm $H_2O$ and over a range of rotational speeds of 1000 to 12,000 RPM, with a leakage area of .01 cm$^2$ (.0017 in.$^2$) and for an outlet taper angle of 60 degrees and an inlet taper angle of 60 degrees, the following flow channel area functions (in cm$^2$) (in.$^2$) were determined to provide a satisfactory approximately linear pressure rise in a gas transport chamber:

$$A_{OC}(t) = .007t^4 + .02t^7 + .007t^{12}$$

$$A_{IC}(t) = .001t^4 + .001t^{12}$$

Selecting widths of the outlet and inlet flow channels of 0.96 cm (0.375 in.) and 0.25 cm (0.10 in.), respectively, the resulting depth profiles for the outlet and inlet flow channels in inches (1 inch = 2,54 cm) are:

$$D_O(t) = .0187t^4 + .0533t^7 + .0187t^{12}$$

$$D_I(t) = .01t^4 + .01t^{12}$$

Another embodiment of the invention is an analytical/numerical method of determining the desired configuration of the blower. This embodiment will be described with reference to Figure 13. In this embodiment, instead of assuming an area function for the outlet and/or inlet flow channels, the desired pressure function is used to analytically and/or numerically calculate the area function that will achieve that desired pressure function. The flow channel dimensions are then selected to achieve the required area function. In one embodiment, only an outlet flow channel is utilized. In one embodiment, the required orifice area is calculated at discrete intervals during the taper time, and the area function is derived from the resulting discrete orifice area values.

In a first step S1, the "taper time" is calculated. As indicated above, the "taper time" is the time that it takes for a rotor lobe to rotate through the selected taper angle. This time may be calculated in similar manner to that described above with respect to the previously described method. A desired iteration time interval and a desired pressure change profile are also selected. For example, a desired iteration time interval may be expressed as a fraction of the taper time, for example, the iteration time interval may be selected to be 1/2000th of the taper time.

In a step S2, the desired flow rates at the outlet orifice areas at a particular normalized time "t" is calculated from the desired rate of change of pressure at that time as specified by the desired pressure change profile. During the first iteration, "t" will be equal to the selected iteration time interval divided by the taper time. During each successive iteration, "t" is incremented by the iteration time interval divided by the taper time. The iterations will continue until "t" equals one (1).

In a step S3, the outlet orifice area required to achieve the desired flow at the current time "t" is calculated using compressible gas flow equations as are known in the art.

At step S4, the value of t is incremented by the iteration time interval, and the process returns to step S2. Steps S2 to S4 are repeated until the value of t reaches 1. The outlet orifice areas calculated for each time t may then be used to construct a plot of desired orifice area versus time, which, turn, can be used to select a outlet flow channel profile (after taking account the clearance leakage area's contribution to the total outlet orifice area).

If a blower according to the present invention is intended to operate over a range of rotational speeds and/or outlet and inlet pressures, the methods of Figures 12 or 13 may be performed at a number of points within the intended operating range, and an average area function/depth profile may be selected from the area functions/depth profiles determined at each of the operating points, or the area function/ depth profile that produces the most satisfactory results over the range of operating points may be selected.

The blower configuration of the invention is advantageous in that it generates substantially less noise than a blower not having the configuration.

It will be understood that the above described arrangements of apparatus and the method therefrom are merely illustrative of applications of the principles of this invention and many other embodiments and modifications may be made without departing from the scope of the invention as defined in the claims.

**Claims**

1. A Roots-type blower with reduced noise emission comprising:

   a housing (22) defining a rotor chamber (28), said rotor chamber comprising an inlet (80) and an outlet (82);
   a first (24) and a second rotor (26) rotably mounted in said chamber (28), each rotor defining a plurality of lobes (70), adjacent lobes of each rotor cooperating with said housing to define at one or more times gas transport chambers (103), said rotors configured to move gas from said inlet (80) via said gas transport chamber (103) to said outlet (82); and
   at least one outlet gas flow channel (90a, 90b) extending from said outlet along an inner surface of said housing at said rotor chamber in a direction opposite a direction of rotation of said rotor, said at least one outlet gas flow channel configured to permit gas to flow from said outlet into a gas transport chamber (103) as said lobes of said rotor rotate towards said outlet,
   **characterised by**:

   said at least one outlet gas flow channel (90a, 90b) configured so that a pressure of said gas in said chamber (28) as said chamber moves towards said outlet changes at an approximately linear rate; or
   said at least one outlet gas flow channel (90a, 90b) defining a flow area which increases generally non-linearly towards the direction of said outlet.

2. The blower in accordance with Claim 1 including:

   at least one outlet gas flow channel (90a, 90b) for each of said rotors, said outlet gas flow channel having a first end (92) and a second end (94), said second end located at said outlet and said first end spaced therefrom in the direction opposite said direction of rotation of said rotor; or
   at least one inlet flow channel (102a, 102b) corresponding to at least one of said rotors (24, 26), said at least one inlet flow channel (102a, 102b) extending from said inlet (80) along an inner surface of said rotor chamber in an opposite direction as the direction of rotation of said rotor, said inlet flow channel configured to permit gas to flow from a chamber to said inlet.

3. The blower in accordance with Claim 1 wherein:

   each outlet gas flow channel (90a,90b) has a cross-sectional area which increases moving in the direction of the first end to the second end thereof; preferably said increase in area is associated with at least an increase in a depth of said channel; or
   said outlet gas flow channel (90a,90b) has a cross-sectional area which increases non-linearly moving in the direction of the first end of the second end thereof.

4. The blower in accordance with Claim 1 wherein:

   said Roots-type blower comprises part of a mechanical ventilator; or
   said rate of change of pressure of said gas varies from linearity by no more than about 10 %; or
   said rate of change of pressure of said gas varies from linearity no more than about 5 %.

5. The blower in accordance with Claim 1 wherein said at least one outlet gas flow channel (90a,90b) defining a flow area which increases generally non-linearly towards the direction of said outlet and a width of said at least one outlet gas flow channel is generally constant and a depth of said at least one channel increases non-linearly towards the direction of said outlet.

6. The blower in accordance with Claim 1 wherein:

   at least one outlet gas flow channel (90a,90b) corresponding to said second rotor (26), said at least one outlet gas flow channel (90a,90b) extending from said outlet (82) along an inner surface of said housing in a direction opposite a direction of rotation of said second rotor, said at least one outlet gas flow channel configured to permit gas to flow from said outlet into a gas transport chamber between two lobes of said second rotor as said lobes of said second rotor rotate towards said outlet; at least one inlet gas flow channel (90a,90b) corresponding to said first rotor (24), said at least one inlet gas flow channel extending from said inlet along an inner surface of said housing at said rotor chamber in a direction of rotation of said first rotor, said at least one inlet gas flow channel (102a,102b) configured to permit gas to flow from said gas transport chamber between two lobes (70) of said first rotor (24) back to said inlet as said lobes of said first rotor rotate towards said outlet; and at least one inlet gas flow channel (102a,102b) corresponding to said second rotor (26), said at least one inlet gas flow channel extending from said inlet along an inner surface of said housing at said rotor chamber (28) in a direction of rotation of said second rotor, said at least one inlet gas flow channel configured to permit gas to flow from said gas transport chamber between two lobes (70) of said second rotor (26) back to said inlet as said lobes of said second rotor rotate towards said outlet.

7. The blower in accordance with Claim 6 wherein:

said inlet and outlet gas flow channels (90a,b; 102a,b) corresponding to said first and second rotors (24,26) are configured such that a net rate of gas flow into said gas transport chambers is approximately constant; or said inlet and outlet gas flow channels corresponding to said first and second rotors are configured to cause an approximately linear rate of pressure change within said gas transport chambers; or said outlet gas flow channels corresponding to said first and second rotors have a cross-sectional area which increases generally non-linearly moving in the direction of said outlet; or said Roots-type blower comprises part of a mechanical ventilator.

8. The blower in accordance with Claim 6 wherein said outlet gas flow channels (90a, 90b) corresponding to said first and second rotors (24, 26) have a cross-sectional area which increases generally non-linearly moving in the direction of said outlet and said outlet gas flow channels corresponding to said first and second rotors have a cross-sectional area which increases continuously moving in the direction of said outlet.

9. The blower in accordance with Claim 6 wherein said inlet and outlet gas flow channels (90a,b; 102a,b) corresponding to said first and second rotors (24, 26) are configured such that a net rate of gas flow into said gas transport chambers is approximately constant and:

said gas flow rate changes by no more than about 10 %; or said gas flow rate changes by no more than about 5 %; or said rate of change of pressure of said gas varies from linearity changes by no more than about 5 %:

10. The blower in accordance with Claim 6 wherein said inlet and outlet gas flow channels (90a,b; 102a,b) corresponding to said first and second rotors (24,26) are configured to cause an approximately linear rate of pressure change within said gas transport chambers and said rate of change of pressure of said gas varies from linearity by no more than about 10 %.

11. A method for configuring a gas flow path for providing a flow of gas between a port of a Roots-type blower and a gas transport chamber formed between lobes of at least one rotor of said blower, comprising the steps of:

selecting a length for said flow path; selecting a desired gas transport chamber function that defines desired values of a characteristic of gas in said gas transport chamber as a function of rotor position; selecting an area function that defines a cross-sectional area of said flow path along said length of said flow path; calculating estimated values of said characteristic of said gas in said gas transport chamber corresponding to said area function; comparing said estimated values to said desired values; repeating said steps of selecting an area function, calculating estimated values, and comparing said estimated values to said desired values until said estimated values are approximately equal to said desired ,values wherein at least one outlet gas flow channel (90a, 90b) extending from the outlet of a blower rotor chamber (28) is configured so that a pressure of said gas in said chamber (28) changes at an approximately linear rats as said chamber moves towards said outlet; or said at least one outlet gas flow channel (90a, 90b) defines a flow area which increases generally non-linearly towards the direction of said outlet.

12. The method of claim 11 wherein said length of said flow path comprises a taper angle; preferably said rotor position is represented by a taper time.

13. The method of claim 11 wherein said characteristic of said gas in said gas transport chamber comprises a pressure of said gas; preferably said desired gas transport chamber function comprises an approximately linear rate of change in pressure of gas in said gas transport chamber.

14. The method of claim 11 wherein said characteristic of said gas in said gas transport chamber comprises a flow rate of gas into said gas transport chamber; preferably said desired gas transport chamber function comprises an approximately constant rate of gas flow to said gas transport chamber.

15. The method of claim 11 wherein said area function comprises a constant component and a variable component; preferably:

said constant component comprises a leakage area; or
said variable component comprises a polynomial.

16. The method of claim 15 wherein said constant component comprises a leakage area and said variable component comprises a polynomial, where said polynomial comprises a polynomial of the form $Et^4 + Ft^7 + Gt^{12}$ where "E," "F," and "G" are constants and wherein "t" is a normalized taper time.

17. The method of claim 16 wherein E equals approximately:

.044 cm$^2$ (.007 in.$^2$), F equals approximately .126 cm$^2$ (.02 in.$^2$), and G equals approximately .044 cm$^2$ (.007 in.$^2$); or
.006 cm$^2$ (.001 in.$^2$), F equals zero, and G equals approximately .006 cm$^2$ (.001 in.$^2$).

18. The method of claim 11 wherein:

said port comprises an outlet port of said blower; or
said port comprises an inlet port of said blower; or
said Roots-type blower comprises part of a mechanical ventilator.

19. The method of claim 11 further comprising the step of configuring a gas flow channel that corresponds to said area function; preferably said gas flow channel comprises a generally constant width; more preferably a depth of said gas flow channel increases along its length in a generally non-linear manner.

20. The method of claim 19 further comprising the step of configuring a gas flow channel that corresponds to said area function wherein:

said gas flow channel comprises an outlet flow channel; or
said gas flow channel comprises an inlet flow channel.

21. A method for configuring a gas flow path for providing a flow of gas between a port of a Roots-type blower and a gas transport chamber formed between lobes of at least one rotor of said blower, comprising the steps of:

selecting a length for said flow path; selecting a desired gas transport chamber function that defines desired values of a characteristic of gas in said gas transport chamber as a function of rotor position;
selecting an initial incremental rotor position;
calculating an initial desired cross-sectional flow area corresponding to said gas transport chamber function at said initial incremental rotor position;
selecting a succeeding incremental rotor position;
calculating a succeeding desired cross-sectional flow area corresponding to said gas transport chamber function at said succeeding incremental rotor position;
repeating said steps of selecting a succeeding incremental rotor position and calculating a succeeding desired cross-sectional flow area for rotor positions traversing said length of said flow path,
wherein
at least one outlet gas flow channel (90a, 90b) extending from the outlet of a blower rotor chamber (28) is configured so that a pressure of said gas in said chamber (28) changes at an approximately linear rate as said chamber moves towards said outlet; or said at least one outlet gas flow channel (90a, 90b) a flow area which increases generally non-linearly towards the direction of said outlet.

**22.** The method of claim 21 wherein said length of said flow path comprises a taper angle; preferably said rotor position is represented by a taper time.

**23.** The method of claim 21 wherein said characteristic of said gas in said gas transport chamber comprises a pressure of said gas; preferably said desired gas transport chamber function comprises an approximately linear rate of change in pressure of gas in said gas transport chamber.

**24.** The method of claim 21 wherein said characteristic of said gas in said gas transport chamber comprises a flow rate of gas into said gas transport chamber; preferably said desired gas transport chamber function comprises an approximately constant rate of gas flow to said gas transport chamber.

**25.** The method of claim 21 wherein:

said port comprises an outlet port of said blower; or
said port comprises an inlet port of said blower.

**26.** The method of claim 21 further comprising the step of configuring a gas flow channel that corresponds to said desired cross sectional flow areas.

**27.** The method of claim 26 wherein:

said gas flow channel comprises a generally constant width; or
said gas flow channel comprises a generally constant width and a depth of said gas flow channel increases along its length in a generally non-linear manner; or
said gas flow channel comprises an outlet flow channel; or
said gas flow channel comprises an inlet flow channel.

**28.** The method of claim 21 wherein said Roots-type blower comprises part of a mechanical ventilator.

**Patentansprüche**

**1.** Gebläse vom Roots-Typ mit verringerter Geräuschemission, wobei das Gebläse umfasst:

ein Gehäuse (22), das eine Rotorkammer (28) definiert, wobei die genannte Rotorkammer einen Einlass (80) und einen Auslass (82) umfasst;
einen ersten (24) und eine zweiten (26) Rotor, die drehbar in die genannte Kammer (28) eingebaut sind, wobei jeder Rotor eine Mehrzahl von Lappen (70) definiert, wobei benachbarte Lappen jedes Rotors mit dem genannten Gehäuse zusammenwirken, um ein- oder mehrmals Gastransportkammern (103) zu definieren, wobei die genannten Rotoren so konfiguriert sind, dass sie Gas von dem genannten Einlass (80) über die genannte Gastransportkammer (103) zu dem genannten Auslass (82) bewegen; und
wenigstens einen Auslass-Gasströmungskanal (90a, 90b), der von dem genannten Auslass entlang einer Innenoberfläche des genannten Gehäuses bei der genannten Rotorkammer in einer Richtung, die zu einer Drehrichtung des genannten Rotors entgegengesetzt ist, verläuft, wobei der genannte wenigstens eine Auslass-Gasströmungskanal so konfiguriert ist, dass er zulässt, dass Gas von dem genannten Auslass in eine Gastransportkammer (103) strömt, während sich die genannten Lappen des genannten Rotors in Richtung des genannten Auslasses drehen,
**dadurch gekennzeichnet, dass**
der genannte wenigstens eine Auslass-Gasströmungskanal (90a, 90b) so konfiguriert ist, dass ein Druck des genannten Gases in der genannten Kammer (28), während sich die genannte Kammer in Richtung des genannten Auslasses bewegt, mit einer näherungsweise linearen Rate ändert; oder
der genannte wenigstens eine Auslass-Gasströmungskanal (90a, 90b) einen Durchflussquerschnitt definiert, der in Richtung des genannten Auslasses allgemein nichtlinear zunimmt.

**2.** Gebläse gemäß Anspruch 1, das enthält:

wenigstens einen Auslass-Gasströmungskanal (90a, 90b) für jeden der genannten Rotoren, wobei der genannte Auslass-Gasströmungskanal ein erstes Ende (92) und ein zweites Ende (94) aufweist, wobei sich das genannte zweite Ende bei dem genannten Auslass und das genannte erste Ende in einer Richtung, die zu der genannten Drehrichtung des genannten Rotors entgegengesetzt ist, davon beabstandet befindet; oder
wenigstens einen Einlassströmungskanal (102a, 102b), der wenigstens einem der genannten Rotoren (24, 26) entspricht, wobei der genannte wenigstens eine Einlassströmungskanal (102a, 102b) von dem genannten Einlass (80) entlang einer Innenoberfläche der genannten Rotorkammer in einer Richtung, die zu der Drehrichtung des genannten Rotors entgegengesetzt ist, verläuft, wobei der genannte Einlassströmungskanal so konfiguriert ist, dass er zulässt, dass Gas von einer Kammer zu dem genannten Einlass strömt.

3. Gebläse gemäß Anspruch 1, bei dem:

jeder Auslass-Gasströmungskanal (90a, 90b) eine Querschnittsfläche aufweist, die bei Bewegung in Richtung von dem ersten Ende zu dem zweiten Ende davon zunimmt; wobei die genannte Zunahme der Fläche vorzugsweise wenigstens einer Zunahme einer Tiefe des genannten Kanals zugeordnet ist; oder
der genannte Auslass-Gasströmungskanal (90a, 90b) eine Querschnittsfläche aufweist, die bei Bewegung in Richtung von dem ersten Ende von dem zweiten Ende davon nichtlinear zunimmt.

4. Gebläse gemäß Anspruch 1, bei dem:

das genannte Gebläse vom Roots-Typ einen Teil eines mechanischen Lüfters umfasst; oder
die genannte Druckänderungsrate des genannten Gases um nicht mehr als etwa 10 % von der Linearität abweicht; oder
die genannte Druckänderungsrate des genannten Gases nicht mehr als etwa 5 % von der Linearität abweicht.

5. Gebläse gemäß Anspruch 1, bei dem der genannte wenigstens eine Auslass-Gasströmungskanal (90a, 90b) einen Durchflussquerschnitt definiert, der in Richtung des genannten Auslasses allgemein nichtlinear zunimmt, und bei dem eine Breite des genannten wenigstens einen Auslass-Gasströmungskanals allgemein konstant ist und eine Tiefe des genannten wenigstens einen Kanals in Richtung des genannten Auslasses nichtlinear zunimmt.

6. Gebläse gemäß Anspruch 1, bei dem:

wenigstens ein Auslass-Gasströmungskanal (90a, 90b) dem genannten zweiten Rotor (26) entspricht, wobei der genannte wenigstens eine Auslass-Gasströmungskanal (90a, 90b) von dem genannten Auslass (82) entlang einer Innenoberfläche des genannten Gehäuses in einer Richtung, die zu einer Drehrichtung des genannten zweiten Rotors entgegengesetzt ist, verläuft, wobei der genannte wenigstens eine Auslass-Gasströmungskanal so konfiguriert ist, dass er zulässt, dass Gas von dem genannten Auslass in eine Gastransportkammer zwischen zwei Lappen des genannten zweiten Rotors strömt, während sich die genannten Lappen des genannten zweiten Rotors in Richtung des genannten Auslasses drehen; wenigstens ein Einlass-Gasströmungskanal (90a, 90b) dem genannten ersten Rotor (24) entspricht, wobei der genannte wenigstens eine Einlass-Gasströmungskanal von dem genannten Einlass entlang einer Innenoberfläche des genannten Gehäuses bei der genannten Rotorkammer in einer Drehrichtung des genannten ersten Rotors verläuft, wobei der genannte wenigstens eine Einlass-Gasströmungskanal (102a, 102b) so konfiguriert ist, dass er zulässt, dass Gas von der genannten Gastransportkammer zwischen zwei Lappen (70) des genannten ersten Rotors (24) zurück zu dem genannten Einlass strömt, während sich die genannten Lappen des genannten ersten Rotors in Richtung des genannten Auslasses drehen; und wenigstens ein Einlass-Gasströmungskanal (102a, 102b) dem genannten zweiten Rotor (26) entspricht, wobei der genannte wenigstens eine Einlass-Gasströmungskanal von dem genannten Einlass entlang einer Innenoberfläche des genannten Gehäuses bei der genannten Rotorkammer (28) in einer Drehrichtung des genannten zweiten Rotors verläuft, wobei der genannte wenigstens eine Einlass-Gasströmungskanal so konfiguriert ist, dass er zulässt, dass Gas von der genannten Gastransportkammer zwischen zwei Lappen (70) des genannten zweiten Rotors (26) zurück zu dem genannten Einlass strömt, während sich die genannten Lappen des genannten zweiten Rotors in Richtung des genannten Auslasses drehen.

7. Gebläse gemäß Anspruch 6, bei dem:

die genannten Einlass- und Auslass-Gasströmungskanäle (90a, b; 102a, b), die dem genannten ersten und dem genannten zweiten Rotor (24, 26) entsprechen, so konfiguriert sind, dass ein Nettogasdurchfluss in die genannten Gastransportkammern näherungsweise konstant ist; oder
die genannten Einlass- und Auslass-Gasströmungskanäle, die dem genannten ersten und dem genannten zweiten Rotor entsprechen, so konfiguriert sind, dass sie eine näherungsweise lineare Druckänderungsrate innerhalb der Gastransportkammern veranlassen; oder

die genannten Auslass-Gasströmungskanäle, die dem genannten ersten und dem genannten zweiten Rotor entsprechen, eine Querschnittsfläche aufweisen, die bei Bewegung in Richtung des genannten Auslasses allgemein nichtlinear zunimmt; oder

das genannte Gebläse vom Roots-Typ einen Teil eines mechanischen Lüfters umfasst.

8. Gebläse gemäß Anspruch 6, bei dem die genannten Auslass-Gasströmungskanäle (90a, 90b), die dem genannten ersten und dem genannten zweiten Rotor (24, 26) entsprechen, eine Querschnittsfläche aufweisen, die bei Bewegung in Richtung des genannten Auslasses allgemein nichtlinear zunimmt, und die genannten Auslass-Gasströmungskanäle, die dem genannten ersten und dem genannten zweiten Rotor entsprechen, eine Querschnittsfläche aufweisen, die bei Bewegung in Richtung des genannten Auslasses ununterbrochen zunimmt.

9. Gebläse gemäß Anspruch 6, bei dem die genannten Einlass- und Auslass-Gasströmungskanäle (90a, b; 102a, b), die dem genannten ersten und dem genannten zweiten Rotor (24, 26) entsprechen, so konfiguriert sind, dass ein Nettogasdurchfluss in die genannten Gastransportkammern näherungsweise konstant ist, und:

sich der genannte Gasdurchfluss um nicht mehr als etwa 10 % ändert; oder
sich der genannte Gasdurchfluss um nicht mehr als etwa 5 % ändert; oder
die genannte Druckänderungsrate des genannten Gases um nicht mehr als etwa 5 % von Linearitätsänderungen abweicht.

10. Gebläse gemäß Anspruch 6, bei dem die genannten Einlass- und Auslass-Gasströmungskanäle (90a, b; 102a, b), die dem genannten ersten und dem genannten zweiten Rotor (24, 26) entsprechen, so konfiguriert sind, dass sie innerhalb der genannten Gastransportkammern eine näherungsweise lineare Druckänderungsrate veranlassen und die genannte Druckänderungsrate des genannten Gases um nicht mehr als etwa 10 % von der Linearität abweicht.

11. Verfahren zum Konfigurieren eines Gasströmungswegs zum Bereitstellen einer Gasströmung zwischen einem Anschluss eines Gebläses vom Roots-Typ und einer Gastransportkammer, die zwischen Lappen wenigstens eines Rotors des genannten Gebläses gebildet ist, wobei das Verfahren die folgenden Schritte umfasst:

Auswählen einer Länge des genannten Strömungswegs;
Auswählen einer Soll-Gastransportkammerfunktion, die Sollwerte einer Eigenschaft von Gas in der genannten Gastransportkammer in Abhängigkeit von der Rotorstellung definiert;
Auswählen einer Flächenfunktion, die eine Querschnittsfläche des genannten Strömungswegs entlang der genannten Länge entlang des genannten Strömungswegs definiert;
Berechnen von Schätzwerten der genannten Eigenschaft des genannten Gases in der genannten Transportkammer, entsprechend der genannten Flächenfunktion;
Vergleichen der genannten Schätzwerte mit den genannten Sollwerten;
Wiederholen der genannten Schritte des Auswählens einer Flächenfunktion, des Berechnens von Schätzwerten und des Vergleichens der genannten Schätzwerte mit den genannten Sollwerten, bis die genannten Schätzwerte näherungsweise gleich den genannten Sollwerten sind,
wobei
wenigstens ein Auslass-Gasströmungskanal (90a, 90b), der von dem Auslass einer Gebläserotorkammer (28) ausgeht, so konfiguriert ist, dass sich ein Druck des genannten Gases in der genannten Kammer (28) bei Bewegung der genannten Kammer in Richtung des genannten Auslasses mit einer näherungsweise linearen Rate ändert; oder
der genannte wenigstens eine Auslass-Gasströmungskanal (90a, 90b) einen Durchflussquerschnitt definiert, der in Richtung des genannten Auslasses allgemein nichtlinear zunimmt.

12. Verfahren gemäß Anspruch 11, bei dem die genannte Länge des genannten Strömungswegs einen Neigungswinkel umfasst; wobei die genannte Rotorstellung vorzugsweise durch eine Neigungszeit dargestellt ist.

13. Verfahren gemäß Anspruch 11, bei dem die genannte Eigenschaft des genannten Gases in der genannten Gastransportkammer einen Druck des genannten Gases umfasst; wobei die genannte Soll-Gastransportkammerfunktion vorzugsweise eine näherungsweise lineare Änderungsrate des Gasdrucks in der genannten Gastransportkammer umfasst.

14. Verfahren gemäß Anspruch 11, bei dem die genannte Eigenschaft des genannten Gases in der genannten Gastransportkammer einen Gasdurchfluss in die genannte Gastransportkammer umfasst; wobei die genannte Soll-Gastransportkammerfunktion vorzugsweise einen näherungsweise konstanten Gasdurchfluss zu der genannten Gastransportkammer umfasst.

15. Verfahren gemäß Anspruch 11, bei dem die genannte Flächenfunktion eine konstante Komponente und eine variable Komponente umfasst; wobei vorzugsweise:

die genannte konstante Komponente eine Leckfläche umfasst; oder
die genannte variable Komponente ein Polynom umfasst.

16. Verfahren gemäß Anspruch 15, bei dem die genannte konstante Komponente eine Leckfläche umfasst und die genannte variable Komponente ein Polynom umfasst, wobei das genannte Polynom ein Polynom der Form $Et^4 + Ft^7 + Gt^{12}$ umfasst, wobei "E", "F" und "G" Konstanten sind und wobei "t" eine normierte Neigungszeit ist.

17. Verfahren gemäß Anspruch 16, bei dem E näherungsweise gleich:

$0,044\ cm^2$ ($0,007\ Zoll^2$) ist, F näherungsweise gleich $0,126\ cm^2$ ($0,02\ Zoll^2$) ist und G näherungsweise gleich $0,044\ cm^2$ ($0,007\ Zoll^2$) ist; oder
$0,006\ cm^2$ ($0,001\ Zoll^2$) ist, F gleich null ist und G näherungsweise gleich $0,006\ cm^2$ ($0,001\ Zoll^2$) ist.

18. Verfahren gemäß Anspruch 11, bei dem:

der genannte Anschluss einen Auslassanschluss des genannten Gebläses umfasst; oder
der genannte Anschluss einen Einlassanschluss des genannten Gebläses umfasst; oder
das genannte Gebläse vom Roots-Typ einen Teil eines mechanischen Lüfters umfasst.

19. Verfahren gemäß Anspruch 11, das ferner den Schritt des Konfigurierens eines Gasströmungskanals umfasst, der der genannten Flächenfunktion entspricht; wobei der genannte Gasströmungskanal vorzugsweise eine allgemein konstante Breite umfasst; wobei bevorzugter eine Tiefe des genannten Gasströmungskanals entlang seiner Länge auf allgemein nichtlineare Weise zunimmt.

20. Verfahren gemäß Anspruch 19, das ferner den Schritt des Konfigurierens eines Gasströmungskanals umfasst, der der genannten Flächenfunktion entspricht, wobei:

der genannte Gasströmungskanal einen Auslassströmungskanal umfasst; oder
der genannte Gasströmungskanal einen Einlassströmungskanal umfasst.

21. Verfahren zum Konfigurieren eines Gasströmungswegs zum Bereitstellen einer Gasströmung zwischen einem Anschluss eines Gebläses vom Roots-Typ und einer Gastransportkammer, die zwischen Lappen wenigstens eines Rotors des genannten Gebläses gebildet ist, wobei das Verfahren die folgenden Schritte umfasst:

Auswählen einer Länge für den genannten Strömungsweg; Auswählen einer Soll-Gastransportkammerfunktion, die die Sollwerte einer Eigenschaft des Gases in der genannten Gastransportkammer in Abhängigkeit von der Rotorstellung definiert;
Auswählen einer anfänglichen inkrementellen Rotorstellung;
Berechnen einer anfänglichen Soll-Querschnittsfläche, die der genannten Gastransportkammerfunktion entspricht, bei der genannten anfänglichen inkrementellen Rotorstellung;
Auswählen einer nachfolgenden inkrementellen Rotorstellung;
Berechnen einer nachfolgenden Soll-Durchflussquerschnitts, der der genannten Gastransportkammerfunktion entspricht, bei der genannten nachfolgenden inkrementellen Rotorstellung;
Wiederholen der genannten Schritte des Auswählens einer nachfolgenden inkrementellen Rotorstellung und des Berechnens eines nachfolgenden Soll-Durchflussquerschnitts für Rotorstellungen, die die genannte Länge des genannten Strömungswegs überspannen,
wobei
wenigstens ein Auslass-Gasströmungskanal (90a, 90b), der von dem Auslass einer Gebläserotorkammer (28) ausgeht, so konfiguriert ist, dass sich ein Druck des genannten Gases in der genannten Kammer (28) bei Bewegung der genannten Kammer in Richtung des genannten Auslasses mit einer näherungsweise linearen Rate ändert; oder

der genannte wenigstens eine Auslass-Gasströmungskanal (90a, 90b) einen Durchflussquerschnitt definiert, der in Richtung des genannten Auslasses allgemein nichtlinear zunimmt.

22. Verfahren gemäß Anspruch 21, bei dem die genannte Länge des genannten Strömungswegs einen Neigungswinkel umfasst; wobei die genannte Rotorstellung vorzugsweise durch eine Neigungszeit dargestellt wird.

23. Verfahren gemäß Anspruch 21, bei dem die genannte Eigenschaft des genannten Gases in der genannten Gastransportkammer einen Druck des genannten Gases umfasst; wobei die genannte Soll-Gastransportkammerfunktion vorzugsweise eine näherungsweise lineare Änderungsrate des Gasdrucks in der genannten Gastransportkammer umfasst.

24. Verfahren gemäß Anspruch 21, bei dem die genannte Eigenschaft des genannten Gases in der genannten Gastransportkammer einen Gasdurchfluss in die genannte Gastransportkammer umfasst; wobei die genannte Soll-Gastransportkammerfunktion vorzugsweise einen näherungsweise konstanten Gasdurchfluss in die genannte Gastransportkammer umfasst.

25. Verfahren gemäß Anspruch 21, bei dem:

der genannte Anschluss einen Auslassanschluss des genannten Gebläses umfasst; oder
der genannte Anschluss einen Einlassanschluss des genannten Gebläses umfasst.

26. Verfahren gemäß Anspruch 21, das ferner den Schritt des Konfigurierens eines Gasströmungskanals umfasst, der den genannten Soll-Durchflussquerschnitten entspricht.

27. Verfahren gemäß Anspruch 26, bei dem:

der genannte Gasströmungskanal eine allgemein konstante Breite umfasst; oder
der genannte Gasströmungskanal eine allgemein konstante Breite umfasst und eine Tiefe des genannten Gasströmungskanals entlang seiner Länge auf eine allgemein nichtlineare Weise zunimmt; oder
der genannte Gasströmungskanal einen Auslassströmungskanal umfasst; oder
der genannte Gasströmungskanal einen Einlassströmungskanal umfasst.

28. Verfahren gemäß Anspruch 21, bei dem das Gebläse vom Roots-Typ einen Teil eines mechanischen Lüfters umfasst.

## Revendications

1. Soufflante de type Roots avec une émission de bruit réduite, comprenant :

un boîtier (22) définissant une chambre de rotor (28), ladite chambre de rotor comprenant une entrée (80) et une sortie (82) ;
un premier (24) et un second (26) rotor montés de manière rotative dans ladite chambre (28), chaque rotor définissant une pluralité de lobes (70), les lobes adjacents de chaque rotor coopérant avec ledit boîtier afin de définir en une fois ou en plusieurs fois, des chambres de transport de gaz (103), lesdits rotors étant configurés pour déplacer le gaz de ladite entrée (80) via lesdites chambres de transport de gaz (103) jusqu'à ladite sortie (82) ; et
au moins un canal d'écoulement de gaz de sortie (90a, 90b) s'étendant à partir de ladite sortie le long d'une surface interne dudit boîtier au niveau de ladite chambre de rotor dans une direction opposée à une direction de rotation dudit rotor, ledit au moins un canal d'écoulement de gaz de sortir étant configuré pour permettre au gaz de s'écouler à partir de ladite sortie dans une chambre de transport de gaz (103), au fur et à mesure que lesdits lobes dudit rotor tournent vers ladite sortie, **caractérisé par** :
ledit au moins un canal d'écoulement de gaz de sortie (90a, 90b) configuré de sorte qu'une pression dudit gaz dans ladite chambre (28) lorsque ladite chambre se déplace vers ladite sortie, change à une vitesse approximativement linéaire ; ou bien
ledit au moins un canal d'écoulement de gaz de sortie (90a, 90b) définissant une surface d'écoulement qui augmente généralement de manière non linéaire vers la direction de ladite sortie.

2. Soufflante selon la revendication 1, comprenant :

au moins un canal d'écoulement de gaz de sortie (90a, 90b) pour chacun desdits rotors, ledit canal d'écoulement de gaz de sortie ayant une première extrémité (92) et une seconde extrémité (94), ladite seconde extrémité étant positionnée au niveau de ladite sortie et ladite première extrémité étant espacée de celle-ci dans une direction opposée à ladite direction de rotation dudit rotor ; ou bien

au moins un canal d'écoulement d'entrée (102a, 102b) correspondant à au moins l'un desdits rotors (24, 26), ledit au moins un canal d'écoulement d'entrée (102a, 102b) s'étendant à partir de ladite entrée (80) le long d'une surface interne de ladite chambre de rotor dans une direction opposée à la direction de rotation dudit rotor, ledit canal d'écoulement d'entrée étant configuré pour permettre au gaz de s'écouler à partir d'une chambre vers ladite sortie.

**3.** Soufflante selon la revendication 1, dans laquelle :

chaque canal d'écoulement de gaz de sortie (90a, 90b) a une surface transversale qui augmente en se déplaçant dans la direction de la première extrémité jusqu'à sa seconde extrémité ; de préférence ladite augmentation de surface est associée avec au moins une augmentation d'une profondeur dans ledit canal ; ou bien

ledit canal d'écoulement de gaz de sortie (90a, 90b) a une surface transversale qui augmente en se déplaçant de manière non linéaire dans la direction de la première extrémité de sa seconde extrémité.

**4.** Soufflante selon la revendication 1, dans laquelle :

ladite soufflante de type Roots comprend une partie d'un ventilateur mécanique ; ou bien

ladite vitesse de changement de pression dudit gaz varie par rapport à la linéarité selon une valeur non supérieure à environ 10% ; ou bien

ladite vitesse de changement de pression dudit gaz varie par rapport à la linéarité selon une valeur non supérieure à environ 5%.

**5.** Soufflante selon la revendication 1, dans laquelle ledit au moins un canal d'écoulement de gaz de sortie (90a, 90b) définit une surface d'écoulement qui augmente généralement de manière non linéaire vers la direction de ladite sortie et une largeur dudit au moins un canal d'écoulement de gaz de sortie est généralement constante et une profondeur dudit un canal augmente de manière non linéaire vers la direction de ladite sortie.

**6.** Soufflante selon la revendication 1, dans laquelle :

au moins un canal d'écoulement de gaz de sortie (90a, 90b) correspondant audit second rotor (26), ledit au moins un canal d'écoulement de gaz de sortie (90a, 90b) s'étend à partir de ladite sortie (82) le long d'une surface interne dudit boîtier dans une direction opposée à une direction de rotation dudit second rotor, ledit au moins un canal d'écoulement de gaz de sortie étant configuré pour permettre au gaz de s'écouler à partir de ladite sortie dans une chambre de transport de gaz entre deux lobes dudit second rotor lorsque lesdits lobes dudit second rotor tournent vers ladite sortie ; au moins un canal d'écoulement de gaz d'entrée (90a, 90b) correspondant audit premier rotor (24), ledit au moins un canal d'écoulement de gaz d'entrée s'étendant à partir de ladite entrée le long d'une surface interne dudit boîtier au niveau de ladite chambre de rotor dans une direction de rotation dudit premier rotor, ledit au moins un canal d'écoulement de gaz d'entrée (102a, 102b) étant configuré pour permettre au gaz revenir en s'écoulant de ladite chambre de transport de gaz entre deux lobes (70) dudit premier rotor (24) à ladite entrée lorsque lesdits lobes dudit premier rotor tournent vers ladite sortie ; et au moins un canal d'écoulement de gaz d'entrée (102a, 102b) correspondant audit second rotor (26), ledit au moins un canal d'écoulement de gaz d'entrée s'étendant à partir de ladite entrée le long d'une surface interne dudit boîtier au niveau de ladite chambre de rotor (28) dans une direction de rotation dudit second rotor, ledit au moins un canal d'écoulement de gaz d'entrée étant configuré pour permettre au gaz de revenir en s'écoulant de ladite chambre de transport de gaz entre deux lobes (70) dudit second rotor (26) à ladite entrée, lorsque lesdits lobes dudit second rotor tournent vers ladite sortie.

**7.** Soufflante selon la revendication 6, dans laquelle :

lesdits canaux d'écoulement de gaz d'entrée et de sortie (90a, b ; 102a, b) correspondant auxdits premier et second rotors (24, 26) sont configurés de sorte qu'un débit de gaz net dans ladite chambre de transport de gaz est approximativement constant ; ou bien

lesdits canaux d'écoulement de gaz d'entrée et de sortie correspondant auxdits premier et second rotors sont configurés pour provoquer une vitesse de changement de pression approximativement linéaire dans lesdites chambres de transport de gaz ; ou bien
lesdits canaux d'écoulement de gaz de sortie correspondant auxdits premier et second rotors ont une surface transversale qui augmente de manière généralement non linéaire en se déplaçant dans la direction de ladite sortie ; ou bien
ladite soufflante de type Roots comprend une partie de ventilateur mécanique.

8. Soufflante selon la revendication 6, dans laquelle lesdits canaux d'écoulement de gaz de sortie (90a, 90b) correspondant auxdits premier et second rotors (24, 26) ont une surface transversale qui augmente de manière généralement non linéaire en se déplaçant dans la direction de ladite sortie, et les canaux d'écoulement de gaz de sortie correspondant auxdits premier et second rotors ont une surface transversale qui augmente de manière continue en se déplaçant dans la direction de ladite sortie.

9. Soufflante selon la revendication 6, dans laquelle lesdits canaux d'écoulement de gaz d'entrée et de sortie (90a, b ; 102a, b) correspondant auxdits premier et second rotors (24, 26) sont configurés de sorte qu'un débit de gaz net dans lesdites chambres de transport de gaz est approximativement constant ; et
ledit débit de gaz change selon une valeur non supérieure à environ 10% ; ou bien
ledit débit de gaz change selon une valeur non supérieure à environ 5% ; ou bien
ladite vitesse de changement de pression dudit gaz varie par rapport à la linéarité, et change selon une valeur non supérieure à 5%.

10. Soufflante selon la revendication 6, dans laquelle lesdits canaux d'écoulement de gaz d'entrée et de sortie (90a,b ; 102a,b) correspondant auxdits premier et second rotors (24, 26) sont configurés pour provoquer une vitesse de changement de pression approximativement linéaire à l'intérieur desdites chambres de transport de gaz et ladite vitesse de chambre de pression dudit gaz varie par rapport à la linéarité selon une valeur non supérieure à environ 10%.

11. Procédé pour configurer une trajectoire d'écoulement de gaz afin de fournir un écoulement de gaz entre un orifice d'une soufflante de type Roots et une chambre de transport de gaz formée entre des lobes d'au moins un rotor de ladite soufflante, comprenant les étapes consistant à :

sélectionner une longueur pour ladite trajectoire d'écoulement ;
sélectionner une fonction de chambre de transport de gaz souhaitée qui définit des valeurs souhaitées d'une caractéristique du gaz dans ladite chambre de transport de gaz en fonction de la position du rotor ;
sélectionner une fonction de surface qui définit une surface transversale de ladite trajectoire d'écoulement le long de ladite longueur de ladite trajectoire d'écoulement ;
calculer des valeurs estimées de ladite caractéristique dudit gaz dans ladite chambre de transport de gaz correspondant à ladite fonction de surface ;
comparer lesdites valeurs estimées auxdites valeurs souhaitées ;
répéter lesdites étapes consistant à sélectionner une fonction de surface, calculer des valeurs estimées et comparer lesdites valeurs estimées auxdites valeurs souhaitées jusqu'à ce que lesdites valeurs estimées soient approximativement égales auxdites valeurs souhaitées,
dans lequel :

au moins un canal d'écoulement de gaz de sortie (90a, 90b) s'étendant à partir de la sortie d'une chambre de rotor de soufflante (28) est configuré de sorte qu'une pression dudit gaz dans ladite chambre (28) change à un débit approximativement linéaire lorsque ladite chambre se déplace vers ladite sortie ; ou bien
ledit au moins un canal d'écoulement de gaz de sortie (90a, 90b) définit une surface d'écoulement qui augmente de manière généralement non linéaire dans la direction de ladite sortie.

12. Procédé selon la revendication 11, dans lequel ladite longueur de ladite trajectoire d'écoulement comprend un angle de conicité ; de préférence ladite position du rotor est représentée par un temps progressivement rétréci.

13. Procédé selon la revendication 11, dans lequel ladite caractéristique dudit gaz dans ladite chambre de transport de gaz comprend une pression dudit gaz ; de préférence ladite fonction de chambre de transport de gaz souhaitée comprend une vitesse de changement de pression de gaz approximativement linéaire dans ladite chambre de transport de gaz.

14. Procédé selon la revendication 11, dans lequel ladite caractéristique dudit gaz dans ladite chambre de transport de gaz comprend un débit de gaz dans ladite chambre de transport de gaz ; de préférence ladite fonction de chambre de transport de gaz souhaitée comprend un débit de gaz approximativement constant vers ladite chambre de transport de gaz.

15. Procédé selon la revendication 11, dans lequel ladite fonction de surface comprend un composant constant et un composant variable ; de préférence :

ledit composant constant comprend une surface de fuite ; ou bien
ledit composant variable comprend une fonction polynomiale.

16. Procédé selon la revendication 15, dans lequel ledit composant constant comprend une surface de fuite et ledit composant variable comprend une fonction polynomiale, dans lequel ladite fonction polynomiale comprend une fonction polynomiale de la forme $Et^4 + Ft^7 + Gt^{12}$ où « E », « F » et « G » sont des constantes et où « t » est un temps progressivement rétréci normalisé.

17. Procédé selon la revendication 16, dans lequel E égale approximativement :

$0,044$ cm$^2$ ($0,007$ pouce$^2$), F égale approximativement $0,126$ cm$^2$ ($0,02$ pouce$^2$), et G égale approximativement $0,044$ cm$^2$ ($0,007$ pouce$^2$) ; ou bien
$0,006$ cm$^2$ ($0,001$ pouce$^2$), F égale zéro et G égale approximativement $0,006$ cm$^2$ ($0,001$ pouce$^2$).

18. Procédé selon la revendication 11, dans lequel :

ledit orifice comprend un orifice de sortie de ladite soufflante ; ou bien
ledit orifice comprend un orifice d'entrée de ladite soufflante ; ou bien
ladite soufflante de type Roots comprend une partie d'un ventilateur mécanique.

19. Procédé selon la revendication 11, comprenant en outre l'étape consistant à configurer un canal d'écoulement de gaz qui correspond à ladite fonction de surface ; de préférence, ledit canal d'écoulement de gaz comprend une largeur généralement constante ; encore de préférence une profondeur dudit canal d'écoulement de gaz augmente le long de sa longueur d'une manière généralement non linéaire.

20. Procédé selon la revendication 19, comprenant en outre l'étape consistant à configurer un canal d'écoulement de gaz qui correspond à ladite fonction de surface, dans lequel :

ledit canal d'écoulement de gaz comprend un canal d'écoulement de sortie ; ou bien
ledit canal d'écoulement de gaz comprend un canal d'écoulement d'entrée.

21. Procédé pour configurer une trajectoire d'écoulement de gaz afin de fournir un écoulement de gaz entre un orifice d'une soufflante de type Roots et une chambre de transport de gaz formée entre les lobes d'au moins un rotor de ladite soufflante, comprenant les étapes consistant à :

sélectionner une longueur pour ladite trajectoire d'écoulement ; sélectionner une fonction de chambre de transport de gaz souhaitée qui définit des valeurs souhaitées d'une caractéristique du gaz dans ladite chambre de transport de gaz en fonction de la position du rotor ;
sélectionner une position de rotor incrémentielle initiale ;
calculer une surface d'écoulement transversale souhaitée initiale correspondant à ladite fonction de chambre de transport de gaz dans ladite position de rotor incrémentielle initiale ;
sélectionner une position de rotor incrémentielle successive ;
calculer une surface d'écoulement transversale souhaitée successive correspondant à ladite fonction de chambre de transport de gaz dans ladite position de rotor incrémentielle successive ;
répéter lesdites étapes consistant à sélectionner une position de rotor incrémentielle successive et calculer une surface d'écoulement transversale souhaitée successive pour les positions de rotor traversant ladite longueur de ladite trajectoire d'écoulement ;
dans lequel :

au moins un canal d'écoulement de gaz de sortie (90a, 90b) s'étendant à partir de la sortie d'une chambre de rotor de soufflante (28) est configuré de sorte qu'une pression dudit gaz dans ladite chambre (28) change

23

à une vitesse approximativement linéaire lorsque ladite chambre se déplace vers ladite sortie ; ou bien dans lequel ledit au moins un canal d'écoulement de gaz de sortie (90a, 90b) définit une surface d'écoulement qui augmente de manière généralement non linéaire vers la direction de ladite sortie.

**22.** Procédé selon la revendication 21, dans lequel ladite longueur de ladite trajectoire d'écoulement comprend un angle de conicité ; de préférence ladite position de rotor est représentée par un temps progressivement rétréci.

**23.** Procédé selon la revendication 21, dans lequel ladite caractéristique dudit gaz dans ladite chambre de transport de gaz comprend une pression dudit gaz ; de préférence ladite fonction de chambre de transport de gaz souhaitée comprend une vitesse de changement de pression de gaz approximativement linéaire dans ladite chambre de transport de gaz.

**24.** Procédé selon la revendication 21, dans lequel ladite caractéristique dudit gaz dans ladite chambre de transport de gaz comprend un débit de gaz dans ladite chambre de transport de gaz ; de préférence ladite fonction de chambre de transport de gaz souhaitée comprend un débit de gaz approximativement constant jusqu'à ladite chambre de transport de gaz.

**25.** Procédé selon la revendication 21, dans lequel :

ledit orifice comprend un orifice de sortie de ladite soufflante ; ou bien
ledit orifice comprend un orifice d'entrée de ladite soufflante.

**26.** Procédé selon la revendication 21, comprenant en outre l'étape consistant à configurer un canal d'écoulement de gaz qui correspond auxdites surfaces d'écoulement transversal souhaitées.

**27.** Procédé selon la revendication 26, dans lequel :

ledit canal d'écoulement de gaz comprend une largeur généralement constante ; ou bien
ledit canal d'écoulement de gaz comprend une largeur généralement constante et une profondeur de ladite chambre d'écoulement de gaz augmente le long de sa longueur d'une manière généralement non linéaire ; ou bien
ledit canal d'écoulement de gaz comprend un canal d'écoulement de sortie ; ou bien
ledit canal d'écoulement de gaz comprend un canal d'écoulement d'entrée.

**28.** Procédé selon la revendication 21, dans lequel ladite soufflante de type Roots comprend une partie d'un ventilateur mécanique.

**Flow Rate**

**FIG. 1**
**(Prior Art)**

**Pressure**

**FIG. 2**
**(Prior Art)**

**FIG. 3**

EP 1 773 434 B1

**FIG. 4**

**FIG. 5**

FIG. 6

**FIG. 7**

FIG. 8

FIG. 9

**FIG. 10**

**FIG. 10A**

**FIG. 11A**          **FIG. 11B**

| | |
|---|---|
| Determine taper time | Step S1 |
| Determine orifice areas | Step S2 |
| Calculate air flow rate at orifices | Step S3 |
| Determine pressure changes associated with air flows | Step S4 |

Change parameter(s)   **No** ◄ Pressure change linear ? ► **Yes** Utilize data to create blower

Step S6                Step S5                Step S7

**FIG. 12**

Determine taper time,
Desired pressure function,
and time interval | Step S1

Determine flow rate /
pressure | Step S2

Calculate area | Step S3

Increment + | Step S4

**FIG. 13**

Flow
Rate

t

**FIG. 14**

Pressure

t

**FIG. 15**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 91274704 A
- US 49242103 P
- DE 3238015 A1
- US 5868133 A, DeVries
- US 84769304 A

**Non-patent literature cited in the description**

- **J. D. Anderson.** The Analysis & Design of Pneumatic Systems. Krieger Publishing Co,